(19) Europäisches Patentamt
European Patent Office
Office européen des brevets

(11) **EP 4 710 918 A1**

(12) **EUROPEAN PATENT APPLICATION**
published in accordance with Art. 153(4) EPC

(43) Date of publication:
**18.03.2026 Bulletin 2026/12**

(21) Application number: **24835368.2**

(22) Date of filing: **03.07.2024**

(51) International Patent Classification (IPC):
*A61K 9/00* (2006.01)   *A61K 47/34* (2017.01)
*A61K 47/10* (2017.01)   *A61K 45/00* (2006.01)
*A61P 27/02* (2006.01)

(52) Cooperative Patent Classification (CPC):
**A61K 9/00; A61K 31/58; A61K 45/00; A61K 45/06;
A61K 47/10; A61K 47/34; A61P 27/02**

(86) International application number:
**PCT/CN2024/103354**

(87) International publication number:
**WO 2025/007888 (09.01.2025 Gazette 2025/02)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **05.07.2023 CN 202310821685**

(71) Applicant: **Shenyang Xingqi Pharmaceutical Co.,
Ltd.
Shenyang, Liaoning 110167 (CN)**

(72) Inventors:
• LIU, Jidong
  Shenyang, Liaoning 110167 (CN)
• YANG, Qiang
  Shenyang, Liaoning 110167 (CN)
• LI, Li
  Shenyang, Liaoning 110167 (CN)
• ZHOU, Jia
  Shenyang, Liaoning 110167 (CN)

(74) Representative: **Patent 42
5, rue Dicks
4081 Esch-zur-Alzette (LU)**

(54) **OPHTHALMIC PHARMACEUTICAL COMPOSITION AND PREPARATION METHOD THEREFOR, OPHTHALMIC KIT CONTAINING OPHTHALMIC PHARMACEUTICAL COMPOSITION, AND USE**

(57) An ophthalmic pharmaceutical composition and a preparation method therefor, an ophthalmic kit containing the ophthalmic pharmaceutical composition, and a use of the ophthalmic pharmaceutical composition or the ophthalmic kit in the preparation of a drug for preventing and/or treating an eye disease. The pharmaceutical composition contains or is composed of the following components: 1 part by weight of an active pharmaceutical ingredient, 0.95-1.25 parts by weight of a liquid matrix, and 0.35-0.45 parts by weight of a solid matrix, wherein the particle size of the active pharmaceutical ingredient meets: D90≤50 μm.

Fig. 1

**Description**

This application claims priority to Chinese Patent Application No. 202310821685.5 filed on July 5, 2023, the entire contents of which are incorporated herein by reference as a part of this application.

## FIELD OF THE INVENTION

The present invention belongs to the pharmaceutical field. The present invention specifically relates to an ophthalmic pharmaceutical composition, a method for preparing the same, an ophthalmic kit comprising this ophthalmic pharmaceutical composition, and the use of this ophthalmic pharmaceutical composition or ophthalmic kit in the preparation of a medicament for preventing and/or treating an ocular disease.

## BACKGROUND OF THE INVENTION

Anatomically, the eyeball includes two parts, the anterior segment and the posterior segment, wherein the anterior segment accounts for about 1/3, consisting of the cornea, pupil, iris, aqueous humor, ciliary body, and lens; the posterior segment accounts for about 2/3, consisting of the vitreous body, retina, macula, optic nerve, choroid, and sclera. Abnormalities in any part of the eyeball may cause ocular diseases, wherein ocular diseases such as retinal vasculitis, diabetic retinopathy, proliferative vitreoretinopathy, choroidal neovascularization, age-related macular degeneration, cystoid macular edema, vitreomacular adhesion, macular hole, and the like are main causes of visual impairment.

Currently, ocular surface administration and systemic administration are still the main administration methods for treating posterior segment diseases, but due to the presence of the tear barrier, corneal barrier, and conjunctival barrier, ocular surface administration hardly reaches the posterior segment, and moreover, affected by the blood-aqueous humor barrier and blood-retinal barrier, systemic administration requires large doses and multiple administrations to enable the amount of drug reaching the posterior segment to meet therapeutic needs.

Based on this situation, to increase the amount of drug reaching the posterior segment, periocular injection administration and intravitreal injection administration are gradually adopted, but most drugs have a short half-life in the vitreous body, requiring multiple injection administrations to maintain the amount of drug in the vitreous body conforming to therapeutic requirements, which causes pain to patients, reduces patient compliance, and also increases the risk of intraocular infection.

To solve the problems caused by multiple injection administrations, sustained-release implants have begun to be adopted clinically for treating posterior segment diseases. Sustained-release implants are classified into non-biodegradable type and biodegradable type according to materials. For example, Vitrasert, a ganciclovir implant developed by Australian company pSivida for treating cytomegalovirus retinitis, was approved by FDA for marketing in 1996, which adopts non-biodegradable materials polyvinyl alcohol and ethylene vinyl acetate copolymer as controlled-release materials; in 2005, Retisert, a fluocinolone acetonide implant developed by this company mainly for treating non-infectious uveitis, was approved by FDA for marketing. Retisert adopts non-biodegradable materials polyvinyl alcohol and silicone oil as controlled-release materials. Since non-biodegradable sustained-release implants cannot degrade, which requires to be removed by secondary surgery, to solve this problem, biodegradable implants emerge as the times demand. Commonly used biodegradable sustained-release materials include hydroxypropyl methylcellulose, polylactic acid, polyglycolic acid, and polylactic acid-glycolic acid copolymer. Representative marketed products are Surodex and Ozurdex from Allergan Company. The former uses polylactic acid-glycolic acid copolymer and hydroxypropyl methylcellulose as materials to prepare a dexamethasone implant, which can continuously release dexamethasone for 7-10 days for anti-inflammatory after cataract surgery; the latter uses polylactic acid-glycolic acid copolymer as material to prepare a dexamethasone implant for treating macular edema and uveitis.

However, whether non-biodegradable implants or biodegradable implants, they currently need to be implanted into the eye through surgery or special administration devices (for example, Ozurdex is equipped with a dedicated injection device), the operation is relatively complicated, and also brings certain pain to patients.

Moreover, the adoption of dedicated injection devices causes inconvenient operation and increases treatment cost, imposing a great burden on patients.

Therefore, there is currently a need for an ophthalmic pharmaceutical with a simple administration method and sustained-release effect, which does not require complex administration devices during administration, can be administered by conventional simple injection methods, and is easy, simple, and convenient during injection. The pharmaceutical is simply and conveniently injected in liquid form to the lesion site and achieves a slow release effect.

## SUMMARY OF THE INVENTION

The present invention provides an ophthalmic pharmaceutical composition, which can be injected into the eye by conventional simple injection methods, facilitating administration operation, and can slowly release the active pharmaceutical ingredient in the eye for a long time, and can degrade in the eye. In addition, the ophthalmic pharmaceutical composition has excellent storage stability, the pharmaceutical liquid is uniformly dispersed, without stratification, aggregation, or caking.

In the present invention, the simple injection method means that it can be injected into the eye by injection method through a syringe pre-filled with the ophthalmic pharmaceutical composition, and when injecting, a small force conforming to ergonomic requirements can be used to push the drug for injection. With reference to the standard YBB00112004-2015 regarding pre-filled syringe assemblies (with needles), wherein it stipulates that the starting force for piston sliding performance should not exceed 25 N. With reference to this standard, in the present invention, the starting force for pushing the pre-filled syringe (for example, 1 ml syringe barrel) should preferably not exceed 25 N.

The ophthalmic pharmaceutical composition of the present invention comprises the following components, or consists of the following components:

| | |
|---|---|
| active pharmaceutical ingredient | 1 part by weight, |
| liquid matrix | 0.95-1.25 parts by weight, |
| solid matrix | 0.35-0.45 parts by weight, |

wherein the particle size of the active pharmaceutical ingredient satisfies: $D_{90} \leq 50 \ \mu m$.

The present invention also provides a method for preparing the ophthalmic pharmaceutical composition.

The present invention also provides an ophthalmic kit comprising the ophthalmic pharmaceutical composition.

The present invention also provides the use of the ophthalmic pharmaceutical composition or the ophthalmic kit in the preparation of a medicament for preventing and/or treating an ocular disease.

Specifically, the present invention is achieved by the following:

1. An ophthalmic pharmaceutical composition comprising the following components:

| | |
|---|---|
| active pharmaceutical ingredient | 1 part by weight, |
| liquid matrix | 0.95-1.25 parts by weight, |
| solid matrix | 0.35-0.45 parts by weight, |

wherein the particle size of the active pharmaceutical ingredient satisfies: $D_{90} \leq 50 \ \mu m$.

2. The ophthalmic pharmaceutical composition according to statement 1, wherein the particle size of the active pharmaceutical ingredient satisfies: $1 \ \mu m \leq D_{90} \leq 50 \ \mu m$.

3. The ophthalmic pharmaceutical composition according to any one of statements 1-2, consisting of the active pharmaceutical ingredient, the liquid matrix, and the solid matrix.

4. The ophthalmic pharmaceutical composition according to any one of statements 1-3, wherein:

the active pharmaceutical ingredient is selected from one or a mixture of two or more of antitumor drugs, anti-inflammatory drugs, immunosuppressants, neovascularization inhibitors, and glaucoma therapeutic drugs.

5. The ophthalmic pharmaceutical composition according to any one of statements 1-4, wherein:

the active pharmaceutical ingredient is selected from one or a mixture of two or more of chlorambucil, melphalan, cyclophosphamide, ifosfamide, carmustine, lomustine, semustine, chlorozotocin, cisplatin, carboplatin, oxaliplatin, fluorouracil, mitoxantrone, irinotecan, topotecan, vinblastine, vincristine, vindesine, vinorelbine, etoposide, teniposide, paclitaxel, docetaxel, bleomycin, methotrexate, gemcitabine, capecitabine, hydroxyurea, mitomycin, gefitinib, sunitinib, dexamethasone, dexamethasone acetate, prednisone, prednisone acetate, fluocinolone, fluocinolone acetonide, triamcinolone acetonide, methylprednisolone, methylprednisolone aceponate, halobetasol propionate, cortisone, hydrocortisone, cyclosporine, rapamycin, tacrolimus, mycophenolate mofetil, fujimycin, mizoribine, sulfasalazine, azathioprine, methotrexate, bevacizumab, ranibizumab, pegaptanib sodium, aflibercept, latanoprost, travoprost, bimatoprost, bimatoprost, tafluprost, pilocarpine, atropine, hyoscyamine, betaxolol, metoprolol, bupranolol, metipranolol, propranolol, timolol, befunolol, acetazolamide, dorzolamide, brinzolamide, apraclonidine, brimonidine, dipivefrin, guanethidine, dapiprazole, dasatinib, and pharmaceutically acceptable salts thereof.

6. The ophthalmic pharmaceutical composition according to any one of statements 1-5, wherein:

the active pharmaceutical ingredient is selected from one or a mixture of two or more of dexamethasone, latanoprost, dasatinib, triamcinolone acetonide, pilocarpine, methotrexate, cyclosporine, brimonidine, and pharmaceutically acceptable salts thereof.

7. The ophthalmic pharmaceutical composition according to any one of statements 1-6, wherein:

the liquid matrix is a biodegradable liquid matrix.

8. The ophthalmic pharmaceutical composition according to any one of statements 1-7, wherein:

the liquid matrix is one or a mixture of two or more of diethyl succinate, diethyl tartrate, dimethyl glutarate, glyceryl triacetate, polyethylene glycol, glycerol, propylene glycol.

9. The ophthalmic pharmaceutical composition according to statement 8, wherein:

the polyethylene glycol of the liquid matrix is one or a mixture of two or more of polyethylene glycols having a number average molecular weight of 70-750.

10. The ophthalmic pharmaceutical composition according to any one of statements 8-9, wherein:

the polyethylene glycol of the liquid matrix is one or a mixture of two or more of PEG-200, PEG-300, PEG-400, and PEG-600.

11. The ophthalmic pharmaceutical composition according to any one of statements 1-10, wherein:

the liquid matrix is polyethylene glycol.

12. The ophthalmic pharmaceutical composition according to any one of statements 1-11, wherein:

the liquid matrix is polyethylene glycol PEG-400.

13. The ophthalmic pharmaceutical composition according to any one of statements 1-12, wherein:

the solid matrix is a biodegradable solid matrix.

14. The ophthalmic pharmaceutical composition according to any one of statements 1-13, wherein:

the solid matrix is one or a mixture of two or more of polyethylene glycol, hydroxycarboxylate polymers.

15. The ophthalmic pharmaceutical composition according to any one of statements 1-14, wherein:

the solid matrix is one or a mixture of two or more of polyethylene glycol, polylactic acid, polyglycolic acid, polylactic acid-glycolic acid copolymer.

16. The ophthalmic pharmaceutical composition according to any one of statements 14-15, wherein:

the polyethylene glycol of the solid matrix is one or a mixture of two or more of polyethylene glycols having a number average molecular weight of 800-20,000.

17. The ophthalmic pharmaceutical composition according to any one of statements 14-16, wherein:

the polyethylene glycol of the solid matrix is one or a mixture of two or more of PEG-800, PEG-1000, PEG-1500, PEG-2000, PEG-4000, PEG-6000, PEG-8000, PEG-10000, and PEG-20000.

18. The ophthalmic pharmaceutical composition according to any one of statements 1-17, wherein:

the solid matrix is polylactic acid-glycolic acid copolymer.

19. The ophthalmic pharmaceutical composition according to statement 18, wherein:

in the polylactic acid-glycolic acid copolymer, the mass ratio of units derived from glycolic acid to units derived from lactic acid is 1:0.01-100.

20. The ophthalmic pharmaceutical composition according to statement 18, wherein:

in the polylactic acid-glycolic acid copolymer, the mass ratio of units derived from glycolic acid to units derived from lactic acid is 1:0.02-50.

21. The ophthalmic pharmaceutical composition according to statement 18, wherein:

in the polylactic acid-glycolic acid copolymer, the mass ratio of units derived from glycolic acid to units derived from lactic acid is 1:0.05-20.

22. The ophthalmic pharmaceutical composition according to statement 18, wherein:

in the polylactic acid-glycolic acid copolymer, the mass ratio of units derived from glycolic acid to units derived from lactic acid is 1:0.1-10.

23. The ophthalmic pharmaceutical composition according to statement 18, wherein:

in the polylactic acid-glycolic acid copolymer, the mass ratio of units derived from glycolic acid to units derived from lactic acid is 1:0.2-5.

24. The ophthalmic pharmaceutical composition according to statement 18, wherein:

in the polylactic acid-glycolic acid copolymer, the mass ratio of units derived from glycolic acid to units derived from lactic acid is 1:0.5-4.

25. The ophthalmic pharmaceutical composition according to statement 18, wherein:

in the polylactic acid-glycolic acid copolymer, the mass ratio of units derived from glycolic acid to units derived from lactic acid is 1:1-3.

26. The ophthalmic pharmaceutical composition according to statement 15, wherein:

the hydroxycarboxylate polymer of the solid matrix has a weight average molecular weight of 1,000-600,000.

27. The ophthalmic pharmaceutical composition according to statement 15, wherein:

the hydroxycarboxylate polymer of the solid matrix has a weight average molecular weight of 5,000-200,000.

28. The ophthalmic pharmaceutical composition according to statement 15, wherein:

the hydroxycarboxylate polymer of the solid matrix has a weight average molecular weight of 10,000-20,000.

29. The ophthalmic pharmaceutical composition according to any one of statements 1-28, wherein:

the molecular weight distribution Mw/Mn of the solid matrix is 1.0-10.0.

30. The ophthalmic pharmaceutical composition according to any one of statements 1-29, wherein:

the molecular weight distribution Mw/Mn of the solid matrix is 1.5-5.0.

31. The ophthalmic pharmaceutical composition according to any one of statements 1-30, wherein:

the active pharmaceutical ingredient is triamcinolone acetonide,
the liquid matrix is polyethylene glycol PEG-400;
the solid matrix is polylactic acid-glycolic acid copolymer.

32. The ophthalmic pharmaceutical composition according to any one of statements 1-31, wherein:
the ophthalmic pharmaceutical composition is an ophthalmic pharmaceutical preparation.
33. The ophthalmic pharmaceutical composition according to any one of statements 1-32, wherein:
the ophthalmic pharmaceutical composition is an ophthalmic pharmaceutical injection preparation, or a sustained-release insert or implant.
34. The ophthalmic pharmaceutical composition according to statement 33, wherein:
the ophthalmic pharmaceutical injection preparation is an intraocular injection preparation.
35. The ophthalmic pharmaceutical composition according to statement 33, wherein:
the ophthalmic pharmaceutical injection preparation is a subconjunctival injection preparation, intravitreal injection preparation, periocular injection preparation, retrobulbar injection preparation, or intracameral injection preparation.
36. The ophthalmic pharmaceutical composition according to statement 32, wherein:
the ophthalmic pharmaceutical preparation is a sustained-release preparation.
37. The ophthalmic pharmaceutical composition according to any one of statements 1-36, wherein:
the ophthalmic pharmaceutical composition is filled in a pre-filled syringe.
38. The ophthalmic pharmaceutical composition according to statement 37, wherein:
the fill volume of the ophthalmic pharmaceutical composition is 0.01 ml-10 ml.
39. The ophthalmic pharmaceutical composition according to statement 37, wherein:
the fill volume of the ophthalmic pharmaceutical composition is 0.1 ml-2 ml.
40. The ophthalmic pharmaceutical composition according to any one of statements 1-39, wherein:
the administration amount of the ophthalmic pharmaceutical composition is 1 $\mu$l-100 $\mu$l.
41. The ophthalmic pharmaceutical composition according to any one of statements 1-39, wherein:
the administration amount of the ophthalmic pharmaceutical composition is 2-20 $\mu$l.
42. The ophthalmic pharmaceutical composition according to any one of statements 1-41, wherein:
the sustained-release period of the ophthalmic pharmaceutical composition is 30 days-730 days.
43. The ophthalmic pharmaceutical composition according to any one of statements 1-42, wherein:
the sustained-release period of the ophthalmic pharmaceutical composition is 60-120 days.
44. The ophthalmic pharmaceutical composition according to any one of statements 1-43, wherein:
the administration frequency of the ophthalmic pharmaceutical composition is once every 30 days-730 days.
45. The ophthalmic pharmaceutical composition according to any one of statements 1-43, wherein:
the administration frequency of the ophthalmic pharmaceutical composition is once every 60-120 days.
46. The ophthalmic pharmaceutical composition according to any one of statements 1-45, wherein:
the ophthalmic pharmaceutical composition is in liquid form at room temperature.
47. A method for preparing the ophthalmic pharmaceutical composition according to any one of statements 1-46, comprising:

(1) providing an active pharmaceutical ingredient satisfying the particle size relationship defined in any one of statements 1-2;
(2) mixing the solid matrix with the liquid matrix, to obtain a mixed matrix; and
(3) dispersing the active pharmaceutical ingredient provided in step (1) in the mixed matrix obtained in step (2).

48. The method according to statement 47, wherein:
in step (2), the mixed matrix is obtained by dissolving the solid matrix in the liquid matrix.
49. The method according to any one of statements 47-48, wherein:
the method further comprises a step of pulverizing the active pharmaceutical ingredient before step (1).
50. An ophthalmic kit comprising the ophthalmic pharmaceutical composition according to any one of statements 1-46.
51. The ophthalmic kit according to statement 50, wherein:
the ophthalmic kit comprises a pre-filled syringe, wherein the ophthalmic pharmaceutical composition is filled in the pre-filled syringe.
52. The ophthalmic kit according to statement 51, wherein:
the fill volume of the ophthalmic pharmaceutical composition is 0.01 ml-10 ml.
53. The ophthalmic kit according to statement 51, wherein:
the fill volume of the ophthalmic pharmaceutical composition is 0.1 ml-2 ml.

54. The use of the ophthalmic pharmaceutical composition according to any one of statements 1-46 or the ophthalmic kit according to any one of statements 50-53 in the preparation of a medicament for preventing and/or treating an ocular disease.

When the ophthalmic pharmaceutical composition of the present invention comprises 0.95-1.25 parts by weight of the liquid matrix and 0.35-0.45 parts by weight of the solid matrix relative to 1 part by weight of the active pharmaceutical ingredient, and simultaneously the active pharmaceutical ingredient satisfies the particle size range: $D_{90} \leq 50$ μm, the ophthalmic pharmaceutical composition of the present invention meets the required starting force requirement for injection, can be injected into the eye by injection method, facilitates injection operation, and can slowly release the active pharmaceutical ingredient in the eye for a long time, and can slowly degrade in the eye. Moreover, the ophthalmic pharmaceutical composition of the present invention has good storage stability.

## BRIEF DESCRIPTION OF DRAWINGS

The drawings illustrated herein are used to provide further understanding of the present invention and constitute a part of this application. The illustrative embodiments and their descriptions of the present invention are used to explain the present invention and do not constitute an undue limitation of the present invention. In the drawings:

Fig. 1 is an illustrative graph showing the pharmaceutical in vitro release of ophthalmic preparation Example 1B;
Fig. 2 is an illustrative graph showing the pharmaceutical in vitro release degree of ophthalmic preparation Example 2B;
Fig. 3 is an illustrative graph showing the pharmaceutical in vitro release degree of ophthalmic preparation Comparative Example 1.

## DETAILED DESCRIPTION

In the present invention, unless specifically described otherwise, the term "matrix" refers to a substance that does not react with the active pharmaceutical ingredient and contributes to the exertion of the effect of the active pharmaceutical ingredient, for example, a substance that affects the administration, release, penetration, absorption of the active pharmaceutical ingredient, a substance that can impart form to the active pharmaceutical ingredient, and the like.

In the present invention, unless specifically described otherwise, the term "liquid matrix" refers to a matrix that is in liquid state at room temperature (10-30°C).

In the present invention, unless specifically described otherwise, the term "solid matrix" refers to a matrix that is in solid state at room temperature (10-30°C).

In the present invention, unless specifically described otherwise, even if the term "about" is not used to modify the expression regarding a numerical value, this numerical value should be understood as being modified by "about"; the term "about" includes a deviation of ±5% of the stated numerical value, i.e., for a numerical value a, regardless of whether it is modified by "about", it should be understood as representing a range of a±5%a, i.e., 0.95a to 1.05a.

## Ophthalmic Pharmaceutical Composition

The first aspect of the present invention provides an ophthalmic pharmaceutical composition comprising, or consisting of the following components:

| | |
|---|---|
| active pharmaceutical ingredient | 1 part by weight, |
| liquid matrix | 0.95-1.25 parts by weight, |
| solid matrix | 0.35-0.45 parts by weight, |

wherein the particle size of the active pharmaceutical ingredient satisfies: $D_{90} \leq 50$ μm.

In the present invention, when the term "particle size of the active pharmaceutical ingredient" is used, it means that the active pharmaceutical ingredient is in particulate form.

In some embodiments of the first aspect of the present invention, the particle size of the active pharmaceutical ingredient satisfies: $1$ μm $\leq D_{90} \leq 50$ μm.

In some embodiments of the first aspect of the present invention, the active pharmaceutical ingredient is selected from one or a mixture of two or more of antitumor drugs, anti-inflammatory drugs, immunosuppressants, neovascularization inhibitors, and glaucoma therapeutic drugs.

In some embodiments of the first aspect of the present invention, the active pharmaceutical ingredient is selected from one or a mixture of two or more of chlorambucil, melphalan, cyclophosphamide, ifosfamide, carmustine, lomustine, semustine, chlorozotocin, cisplatin, carboplatin, oxaliplatin, fluorouracil, mitoxantrone, irinotecan, topotecan, vinblastine, vincristine, vindesine, vinorelbine, etoposide, teniposide, paclitaxel, docetaxel, bleomycin, methotrexate, gemcitabine, capecitabine, hydroxyurea, mitomycin, gefitinib, sunitinib, dexamethasone, dexamethasone acetate, prednisone, prednisone acetate, fluocinolone, fluocinolone acetonide, triamcinolone acetonide, methylprednisolone, methylprednisolone aceponate, halobetasol propionate, cortisone, hydrocortisone, cyclosporine, rapamycin, tacrolimus, mycophenolate mofetil, fujimycin, mizoribine, sulfasalazine, azathioprine, methotrexate, bevacizumab, ranibizumab, pegaptanib sodium, aflibercept, latanoprost, travoprost, bimatoprost, bimatoprost, tafluprost, pilocarpine, atropine, hyoscyamine, betaxolol, metoprolol, bupranolol, metipranolol, propranolol, timolol, befunolol, acetazolamide, dorzolamide, brinzolamide, apraclonidine, brimonidine, dipivefrin, guanethidine, dapiprazole, dasatinib, and pharmaceutically acceptable salts thereof.

In some embodiments of the first aspect of the present invention, the active pharmaceutical ingredient is selected from one or a mixture of two or more of dexamethasone, latanoprost, dasatinib, triamcinolone acetonide, pilocarpine, methotrexate, cyclosporine, brimonidine, and pharmaceutically acceptable salts thereof. In the present invention, antitumor drugs include, but are not limited to, chlorambucil, melphalan, cyclophosphamide, ifosfamide, carmustine, lomustine, semustine, chlorozotocin, cisplatin, carboplatin, oxaliplatin, fluorouracil, mitoxantrone, irinotecan, topotecan, vinblastine, vincristine, vindesine, vinorelbine, etoposide, teniposide, paclitaxel, docetaxel, bleomycin, methotrexate, gemcitabine, capecitabine, hydroxyurea, mitomycin, gefitinib, sunitinib.

In the present invention, anti-inflammatory drugs include, but are not limited to, dexamethasone, dexamethasone acetate, prednisone, prednisone acetate, fluocinolone, fluocinolone acetonide, triamcinolone acetonide, methylprednisolone, methylprednisolone aceponate, halobetasol propionate, cortisone, hydrocortisone.

In the present invention, immunosuppressants include, but are not limited to, cyclosporine, rapamycin, tacrolimus, mycophenolate mofetil, fujimycin, mizoribine, sulfasalazine, azathioprine, methotrexate.

In the present invention, neovascularization inhibitors include, but are not limited to, bevacizumab, ranibizumab, pegaptanib sodium, aflibercept.

In the present invention, glaucoma therapeutic drugs include, but are not limited to, latanoprost, travoprost, bimatoprost, bimatoprost, tafluprost, pilocarpine, atropine, hyoscyamine, betaxolol, metoprolol, bupranolol, metipranolol, propranolol, timolol, befunolol, acetazolamide, dorzolamide, brinzolamide, apraclonidine, brimonidine, dipivefrin, guanethidine, dapiprazole.

In the ophthalmic pharmaceutical composition of the present invention, the particle size of the active pharmaceutical ingredient satisfies: $D_{90} \leq 50\ \mu m$, preferably $1\ \mu m \leq D_{90} \leq 50\ \mu m$. For example, the particle size $D_{90}$ of the active pharmaceutical ingredient can be 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 30, 31, 32, 33, 34, 35, 36, 37, 38, 39, 40, 41, 42, 43, 44, 45, 46, 47, 48, 49, 50 $\mu m$, or a range defined by any two thereof. In the present invention, unless specifically described otherwise, the particle size $D_{90}$ is measured by light (laser) scattering method. $D_{90}$ refers to the particle size at which, in the cumulative volume distribution curve of the particles, 90% by volume of the particles have a particle size lower than this size, and 10% by volume of the particles have a particle size higher than this size.

In some embodiments of the first aspect of the present invention, the liquid matrix is a biodegradable liquid matrix.

In some embodiments of the first aspect of the present invention, the liquid matrix is one or a mixture of two or more of diethyl succinate, diethyl tartrate, dimethyl glutarate, glyceryl triacetate, polyethylene glycol, glycerol, propylene glycol.

In some embodiments of the first aspect of the present invention, the liquid matrix is polyethylene glycol, or a mixture of polyethylene glycol and at least one selected from diethyl succinate, diethyl tartrate, dimethyl glutarate, glyceryl triacetate, glycerol, propylene glycol.

In some embodiments of the first aspect of the present invention, the liquid matrix is polyethylene glycol.

In some embodiments of the first aspect of the present invention, the liquid matrix is a mixture of polyethylene glycol and at least one selected from diethyl succinate, diethyl tartrate, dimethyl glutarate, glyceryl triacetate, glycerol, propylene glycol.

In some embodiments of the first aspect of the present invention, the polyethylene glycol of the liquid matrix is one or a mixture of two or more of polyethylene glycols having a number average molecular weight of 70-750 (for example, 190-210, 300, 380-420, 570-630, 100, 200, 400, 500, 600, 700); for example, one or a mixture of two or more of PEG-200, PEG-300, PEG-400, and PEG-600.

In some embodiments of the first aspect of the present invention, the polyethylene glycol of the liquid matrix is PEG-400.

In some embodiments of the first aspect of the present invention, relative to 1 part by weight of the active pharmaceutical ingredient, the amount of the liquid matrix can be 0.95-1.25 parts by weight, for example, the amount of the liquid matrix can be 0.950, 0.955, 0.960, 0.965, 0.970, 0.975, 0.980, 0.985, 0.990, 0.995, 1.000, 1.005, 1.010, 1.015, 1.020, 1.025, 1.030, 1.035, 1.040, 1.045, 1.050, 1.055, 1.060, 1.065, 1.070, 1.075, 1.080, 1.085, 1.090, 1.095, 1.100, 1.105, 1.110, 1.115, 1.120, 1.125, 1.130, 1.135, 1.140, 1.145, 1.150, 1.155, 1.160, 1.165, 1.170, 1.175, 1.180, 1.185, 1.190, 1.195, 1.200, 1.205, 1.210, 1.215, 1.220, 1.225, 1.230, 1.235, 1.240, 1.245, 1.250 parts by weight, or a range defined by any two thereof.

In some embodiments of the first aspect of the present invention, when the liquid matrix is a mixture of polyethylene glycol and at least one selected from diethyl succinate, diethyl tartrate, dimethyl glutarate, glyceryl triacetate, glycerol, propylene glycol, the mass ratio of polyethylene glycol to said at least one selected from diethyl succinate, diethyl tartrate, dimethyl glutarate, glyceryl triacetate, glycerol, propylene glycol can be 1:0.01-100, preferably 1:0.1-10, for example, can be 1:0.01, 1:0.02, 1:0.03, 1:0.04, 1:0.05, 1:0.06, 1:0.07, 1:0.08, 1:0.09, 1:0.10, 1:0.12, 1:0.15, 1:0.20, 1:0.25, 1:0.3, 1:0.4, 1:0.5, 1:0.6, 1:0.7, 1:0.8, 1:0.9, 1:1.0, 1:1.2, 1:1.5, 1:1.8, 1:2.0, 1:2.5, 1:3.0, 1:3.5, 1:4.0, 1:4.5, 1:5.0, 1:6.0, 1:7.0, 1:8.0, 1:10, 1:12, 1:15, 1:20, 1:25, 1:30, 1:40, 1:50, 1:60, 1:70, 1:80, 1:90, 1:100, or a range defined by any two thereof.

In some embodiments of the first aspect of the present invention, the solid matrix is a biodegradable solid matrix.

In some embodiments of the first aspect of the present invention, the solid matrix is one or a mixture of two or more of polyethylene glycol, hydroxycarboxylate polymers.

In some embodiments of the first aspect of the present invention, the hydroxycarboxylate polymer is a homopolymer, a copolymer of hydroxycarboxylic acids, or a mixture thereof.

In some embodiments of the first aspect of the present invention, the hydroxycarboxylic acid can be lactic acid, glycolic acid, or a mixture thereof.

In some embodiments of the first aspect of the present invention, the hydroxycarboxylate polymer is one or a mixture of two or more of polylactic acid (or polylactide), polyglycolic acid (or polyglycolide), polylactic acid-glycolic acid copolymer.

In some embodiments of the first aspect of the present invention, the solid matrix is one or a mixture of two or more of polyethylene glycol, polylactic acid, polyglycolic acid, polylactic acid-glycolic acid copolymer.

In some embodiments of the first aspect of the present invention, the solid matrix is one or a mixture of two or more of polyethylene glycol, polylactic acid, polyglycolic acid, polylactic acid-glycolic acid copolymer.

In some embodiments of the first aspect of the present invention, the solid matrix is polylactic acid-glycolic acid copolymer, or a mixture of polylactic acid-glycolic acid copolymer and at least one selected from polyethylene glycol, polylactic acid, polyglycolic acid.

In some embodiments of the first aspect of the present invention, the solid matrix is polylactic acid-glycolic acid copolymer.

In some embodiments of the first aspect of the present invention, the solid matrix is a mixture of polylactic acid-glycolic acid copolymer and at least one selected from polyethylene glycol, polylactic acid, polyglycolic acid.

In some embodiments of the first aspect of the present invention, the polylactic acid-glycolic acid copolymer is one or a mixture of two or more of random copolymers, block copolymers, alternating copolymers, statistical copolymers, gradient copolymers. In some embodiments of the first aspect of the present invention, in the polylactic acid-glycolic acid copolymer, the mass ratio of units derived from glycolic acid to units derived from lactic acid can be 1:0.01-100, preferably 1:0.02-50, preferably 1:0.05-20, preferably 1:0.1-10, preferably 1:0.2-5, preferably 1:0.5-4, preferably 1:1-3, for example, can be 1:0.01, 1:0.02, 1:0.03, 1:0.04, 1:0.05, 1:0.06, 1:0.07, 1:0.08, 1:0.09, 1:0.10, 1:0.12, 1:0.15, 1:0.20, 1:0.25, 1:0.3, 1:0.4, 1:0.5, 1:0.6, 1:0.7, 1:0.8, 1:0.9, 1:1.0, 1:1.2, 1:1.5, 1:1.8, 1:2.0, 1:2.5, 1:3.0, 1:3.5, 1:4.0, 1:4.5, 1:5.0, 1:6.0, 1:7.0, 1:8.0, 1:10, 1:12, 1:15, 1:20, 1:25, 1:30, 1:40, 1:50, 1:60, 1:70, 1:80, 1:90, 1:100, or a range defined by any two thereof.

In some embodiments of the first aspect of the present invention, the polyethylene glycol of the solid matrix is one or a mixture of two or more of polyethylene glycols having a number average molecular weight of 800-20,000 (for example, 800, 900, 1000, 1200, 1400, 1600, 2000, 2800, 3000, 4000, 5000, 6000, 7000, 8000, 9500, 9000, 10000, 11000, 12000, 13000, 14000, 15000, 16000, 17000, 18000, 19000, 20000, or a range defined by any two thereof), for example, one or a mixture of two or more of PEG-800, PEG-1000, PEG-1500, PEG-2000, PEG-4000, PEG-6000, PEG-8000, PEG-10000, and PEG-20000.

In some embodiments of the first aspect of the present invention, the hydroxycarboxylate polymer of the solid matrix has a weight average molecular weight of 1,000-600,000, preferably 5,000-200,000, for example 10,000-20,000, for example, 1000, 2000, 3000, 4000, 5000, 6000, 7000, 8000, 9000, 10000, 15000, 20000, 25000, 30000, 40000, 50000, 60000, 70000, 80000, 90000, 100000, 150000, 200000, 250000, 300000, 400000, 450000, 500000, 550000, 600000, or a range defined by any two thereof.

In some embodiments of the first aspect of the present invention, the polylactic acid has a weight average molecular weight of 1,000-600,000, preferably 5,000-200,000, for example 10,000-20,000, for example, 1000, 2000, 3000, 4000, 5000, 6000, 7000, 8000, 9000, 10000, 15000, 20000, 25000, 30000, 40000, 50000, 60000, 70000, 80000, 90000, 100000, 150000, 200000, 250000, 300000, 400000, 450000, 500000, 550000, 600000, or a range defined by any two thereof.

In some embodiments of the first aspect of the present invention, the polyglycolic acid has a weight average molecular weight of 1,000-600,000, preferably 5,000-200,000, for example 10,000-20,000, for example, 1000, 2000, 3000, 4000, 5000, 6000, 7000, 8000, 9000, 10000, 15000, 20000, 25000, 30000, 40000, 50000, 60000, 70000, 80000, 90000, 100000, 150000, 200000, 250000, 300000, 400000, 450000, 500000, 550000, 600000, or a range defined by any two thereof.

In some embodiments of the first aspect of the present invention, the polylactic acid-glycolic acid copolymer has a weight average molecular weight of 1,000-600,000, preferably 5,000-200,000, for example 10,000-20,000, for example, 1000, 2000, 3000, 4000, 5000, 6000, 7000, 8000, 9000, 10000, 15000, 20000, 25000, 30000, 40000, 50000, 60000, 70000, 80000, 90000, 100000, 150000, 200000, 250000, 300000, 400000, 450000, 500000, 550000, 600000, or a range defined by any two thereof.

Number average molecular weight (Mn) can be measured by conventional methods, such as End-Group Analysis, Ebullioscopy, Cryoscopy, Vapor Pressure Osmometry, Membrane Osmometry, Gel Permeation Chromatography, and the like. In this application, unless specifically described otherwise, the number average molecular weight is determined by Size Exclusion Chromatography.

Weight average molecular weight (Mw) can be measured by conventional methods, such as Light Scattering, Gel Chromatography, Sedimentation Velocity Ultracentrifugation, Small-Angle X-Ray Diffraction, Gel Permeation Chromatography, and the like. In this application, unless specifically described otherwise, the weight average molecular weight is determined by Size Exclusion Chromatography.

In some embodiments of the first aspect of the present invention, the molecular weight distribution (Mw/Mn) of the solid matrix, for example, polyethylene glycol, or hydroxycarboxylate polymer (such as polylactic acid, polyglycolic acid, polylactic acid-glycolic acid copolymer) is not particularly limited, for example, can be 1.0-10.0, for example 1.5-5.0, for example, 1.0, 1.5, 2.0, 2.5, 3.0, 3.5, 4.0, 4.5, 5.0, 5.5, 6.0, 6.5, 7.0, 7.5, 7.0, 7.5, 8.0, 8.5, 9.0, 9.5, 10.0, or a range defined by any two thereof. In this application, unless specifically described otherwise, the molecular weight distribution is determined by size exclusion chromatography.

In some embodiments of the first aspect of the present invention, relative to 1 part by weight of the active pharmaceutical ingredient, the amount of the solid matrix can be 0.35-0.45 parts by weight, for example, can be 0.350, 0.355, 0.360, 0.365, 0.370, 0.375, 0.380, 0.385, 0.390, 0.395, 0.400, 0.405, 0.410, 0.415, 0.420, 0.425, 0.430, 0.435, 0.440, 0.445, 0.450 parts by weight, or a range defined by any two thereof.

In some embodiments of the first aspect of the present invention, when the solid matrix is a mixture of polylactic acid-glycolic acid copolymer and at least one selected from polyethylene glycol, polylactic acid, polyglycolic acid, the mass ratio of polylactic acid-glycolic acid copolymer to said at least one selected from polyethylene glycol, polylactic acid, polyglycolic acid can be 1:0.01-100, preferably 1:0.1-10, for example, can be 1:0.01, 1:0.02, 1:0.03, 1:0.04, 1:0.05, 1:0.06, 1:0.07, 1:0.08, 1:0.09, 1:0.10, 1:0.12, 1:0.15, 1:0.20, 1:0.25, 1:0.3, 1:0.4, 1:0.5, 1:0.6, 1:0.7, 1:0.8, 1:0.9, 1:1.0, 1:1.2, 1:1.5, 1:1.8, 1:2.0, 1:2.5, 1:3.0, 1:3.5, 1:4.0, 1:4.5, 1:5.0, 1:6.0, 1:7.0, 1:8.0, 1:10, 1:12, 1:15, 1:20, 1:25, 1:30, 1:40, 1:50, 1:60, 1:70, 1:80, 1:90, 1:100, or a range defined by any two thereof.

In a preferred embodiment of the first aspect of the present invention, the ophthalmic pharmaceutical composition comprises, or consists of the following components:

active pharmaceutical ingredient; preferably, the active pharmaceutical ingredient is selected from one or a mixture of two or more of antitumor drugs, anti-inflammatory drugs, immunosuppressants, neovascularization inhibitors, and glaucoma therapeutic drugs; preferably, the active pharmaceutical ingredient is selected from one or a mixture of two or more of chlorambucil, melphalan, cyclophosphamide, ifosfamide, carmustine, lomustine, semustine, chlorozotocin, cisplatin, carboplatin, oxaliplatin, fluorouracil, mitoxantrone, irinotecan, topotecan, vinblastine, vincristine, vindesine, vinorelbine, etoposide, teniposide, paclitaxel, docetaxel, bleomycin, methotrexate, gemcitabine, capecitabine, hydroxyurea, mitomycin, gefitinib, sunitinib, dexamethasone, dexamethasone acetate, prednisone, prednisone acetate, fluocinolone, fluocinolone acetonide, triamcinolone acetonide, methylprednisolone, methylprednisolone aceponate, halobetasol propionate, cortisone, hydrocortisone, cyclosporine, rapamycin, tacrolimus, mycophenolate mofetil, fujimycin, mizoribine, sulfasalazine, azathioprine, methotrexate, bevacizumab, ranibizumab, pegaptanib sodium, aflibercept, latanoprost, travoprost, bimatoprost, bimatoprost, tafluprost, pilocarpine, atropine, hyoscyamine, betaxolol, metoprolol, bupranolol, metipranolol, propranolol, timolol, befunolol, acetazolamide, dorzolamide, brinzolamide, apraclonidine, brimonidine, dipivefrin, guanethidine, dapiprazole, dasatinib, and pharmaceutically acceptable salts thereof; preferably, the active pharmaceutical ingredient is selected from one or a mixture of two or more of dexamethasone, latanoprost, dasatinib, triamcinolone acetonide, pilocarpine, methotrexate, cyclosporine, brimonidine, and pharmaceutically acceptable salts thereof; preferably, the active pharmaceutical ingredient is triamcinolone acetonide;

liquid matrix, said liquid matrix being polyethylene glycol, or a mixture of polyethylene glycol and at least one selected from diethyl succinate, diethyl tartrate, dimethyl glutarate, glyceryl triacetate, glycerol, propylene glycol; preferably, when the liquid matrix is a mixture of polyethylene glycol and at least one selected from diethyl succinate, diethyl tartrate, dimethyl glutarate, glyceryl triacetate, glycerol, propylene glycol, the mass ratio of polyethylene glycol to said at least one selected from diethyl succinate, diethyl tartrate, dimethyl glutarate, glyceryl triacetate, glycerol, propylene glycol is 1:0.01-100, preferably 1:0.1-10; preferably said liquid matrix is polyethylene glycol; preferably said polyethylene glycol is one or a mixture of two or more of polyethylene glycols having a number average molecular weight of 70-750 (for example, 190-210, 300, 380-420, 570-630, 100, 200, 400, 500, 600, 700, or a range defined by any two thereof); for example, one or a mixture of two or more of PEG-200, PEG-300, PEG-400, and PEG-600; preferably, said polyethylene glycol is PEG-400;

solid matrix, said solid matrix being polylactic acid-glycolic acid copolymer, or a mixture of polylactic acid-glycolic acid copolymer and at least one selected from polyethylene glycol, polylactic acid, polyglycolic acid; preferably, when the solid matrix is a mixture of polylactic acid-glycolic acid copolymer and at least one selected from polyethylene glycol, polylactic acid, polyglycolic acid, the mass ratio of polylactic acid-glycolic acid copolymer to said at least one selected from polyethylene glycol, polylactic acid, polyglycolic acid can be 1:0.01-100, preferably 1:0.1-10; preferably, in the polylactic acid-glycolic acid copolymer, the mass ratio of units derived from glycolic acid to units derived from lactic acid is 1:0.01-100, preferably 1:0.02-50, preferably 1:0.05-20, preferably 1:0.1-10, preferably 1:0.2-5, preferably 1:0.5-4, preferably 1:1-3; preferably, said solid matrix is polylactic acid-glycolic acid copolymer;

wherein,

relative to 1 part by weight of the active pharmaceutical ingredient, the amount of the liquid matrix is 0.95-1.25 parts by weight, preferably 1.0-1.125 parts by weight,

relative to 1 part by weight of the active pharmaceutical ingredient, the amount of the solid matrix is 0.35-0.45 parts by weight, preferably 0.375-0.40 parts by weight,

the particle size of the active pharmaceutical ingredient satisfies: $D_{90} \leq 50 \ \mu m$, preferably $1 \ \mu m \leq D_{90} \leq 50 \ \mu m$.

In a further preferred embodiment of the first aspect of the present invention, the ophthalmic pharmaceutical composition comprises, or consists of the following components:

active pharmaceutical ingredient; preferably, the active pharmaceutical ingredient is selected from one or a mixture of two or more of antitumor drugs, anti-inflammatory drugs, immunosuppressants, neovascularization inhibitors, and glaucoma therapeutic drugs; preferably, the active pharmaceutical ingredient is selected from one or a mixture of two or more of chlorambucil, melphalan, cyclophosphamide, ifosfamide, carmustine, lomustine, semustine, chlorozotocin, cisplatin, carboplatin, oxaliplatin, fluorouracil, mitoxantrone, irinotecan, topotecan, vinblastine, vincristine, vindesine, vinorelbine, etoposide, teniposide, paclitaxel, docetaxel, bleomycin, methotrexate, gemcitabine, capecitabine, hydroxyurea, mitomycin, gefitinib, sunitinib, dexamethasone, dexamethasone acetate, prednisone, prednisone acetate, fluocinolone, fluocinolone acetonide, triamcinolone acetonide, methylprednisolone, methylprednisolone aceponate, halobetasol propionate, cortisone, hydrocortisone, cyclosporine, rapamycin, tacrolimus, mycophenolate mofetil, fujimycin, mizoribine, sulfasalazine, azathioprine, methotrexate, bevacizumab, ranibizumab, pegaptanib sodium, aflibercept, latanoprost, travoprost, bimatoprost, bimatoprost, tafluprost, pilocarpine, atropine, hyoscyamine, betaxolol, metoprolol, bupranolol, metipranolol, propranolol, timolol, befunolol, acetazolamide, dorzolamide, brinzolamide, apraclonidine, brimonidine, dipivefrin, guanethidine, dapiprazole, dasatinib, and pharmaceutically acceptable salts thereof; preferably, the active pharmaceutical ingredient is selected from one or a mixture of two or more of dexamethasone, latanoprost, dasatinib, triamcinolone acetonide, pilocarpine, methotrexate, cyclosporine, brimonidine, and pharmaceutically acceptable salts thereof; preferably, the active pharmaceutical ingredient is triamcinolone acetonide;

liquid matrix, said liquid matrix being polyethylene glycol; preferably, said polyethylene glycol is one or a mixture of two or more of polyethylene glycols having a number average molecular weight of 70-750 (for example, 190-210, 300, 380-420, 570-630, 100, 200, 400, 500, 600, 700, or a range defined by any two thereof); for example, one or a mixture of two or more of PEG-200, PEG-300, PEG-400, and PEG-600; preferably, said polyethylene glycol is PEG-400; and

solid matrix, said solid matrix being polylactic acid-glycolic acid copolymer; preferably, in the polylactic acid-glycolic acid copolymer, the mass ratio of units derived from glycolic acid to units derived from lactic acid is 1:0.01-100, preferably 1:0.02-50, preferably 1:0.05-20, preferably 1:0.1-10, preferably 1:0.2-5, preferably 1:0.5-4, preferably 1:1-3;

wherein,

relative to 1 part by weight of the active pharmaceutical ingredient, the amount of the liquid matrix is 0.95-1.25 parts by weight, preferably 1.0-1.125 parts by weight,

relative to 1 part by weight of the active pharmaceutical ingredient, the amount of the solid matrix is 0.35-0.45 parts by weight, preferably 0.375-0.40 parts by weight,

the particle size of the active pharmaceutical ingredient satisfies: $D_{90} \leq 50 \ \mu m$, preferably $1 \ \mu m < D_{90} \leq 50 \ \mu m$.

In a further preferred embodiment of the first aspect of the present invention, the ophthalmic pharmaceutical composition comprises, or consists of the following components:

active pharmaceutical ingredient, which is one or a mixture of two or more of dexamethasone, latanoprost, dasatinib, triamcinolone acetonide, pilocarpine, methotrexate, cyclosporine, brimonidine, and pharmaceutically acceptable salts thereof, preferably triamcinolone acetonide;

liquid matrix, said liquid matrix being polyethylene glycol; preferably said polyethylene glycol is one or a mixture of two or more of polyethylene glycols having a number average molecular weight of 70-750 (for example, 190-210, 300, 380-420, 570-630, 100, 200, 400, 500, 600, 700, or a range defined by any two thereof); for example, one or a mixture of two or more of PEG-200, PEG-300, PEG-400, and PEG-600; preferably, said polyethylene glycol is PEG-400; and

solid matrix, said solid matrix being polylactic acid-glycolic acid copolymer, preferably, in the polylactic acid-glycolic acid copolymer, the mass ratio of units derived from glycolic acid to units derived from lactic acid is 1:0.01-100, preferably 1:0.02-50, preferably 1:0.05-20, preferably 1:0.1-10, preferably 1:0.2-5, preferably 1:0.5-4, preferably 1:1-3;

wherein,

relative to 1 part by weight of the active pharmaceutical ingredient, the amount of the liquid matrix is 0.95-1.25 parts by weight, preferably 1.0-1.125 parts by weight,

relative to 1 part by weight of the active pharmaceutical ingredient, the amount of the solid matrix is 0.35-0.45 parts by weight, preferably 0.375-0.40 parts by weight,

the particle size of the active pharmaceutical ingredient satisfies: $D_{90} \leq 50 \ \mu m$, preferably $1 \ \mu m < D_{90} \leq 50 \ \mu m$.

In an even further preferred embodiment of the first aspect of the present invention, the ophthalmic pharmaceutical composition comprises, or consists of the following components:

active pharmaceutical ingredient, which is one or a mixture of two or more of dexamethasone, latanoprost, dasatinib, triamcinolone acetonide, pilocarpine, methotrexate, cyclosporine, brimonidine, and pharmaceutically acceptable salts thereof, preferably triamcinolone acetonide;

liquid matrix, said liquid matrix being polyethylene glycol PEG-400; and

solid matrix, said solid matrix being polylactic acid-glycolic acid copolymer, preferably, in the polylactic acid-glycolic acid copolymer, the mass ratio of units derived from glycolic acid to units derived from lactic acid is 1:0.01-100, preferably 1:0.02-50, preferably 1:0.05-20, preferably 1:0.1-10, preferably 1:0.2-5, preferably 1:0.5-4, preferably 1:1-3;

wherein,

relative to 1 part by weight of the active pharmaceutical ingredient, the amount of the liquid matrix is 0.95-1.25 parts by weight, preferably 1.0-1.125 parts by weight,

relative to 1 part by weight of the active pharmaceutical ingredient, the amount of the solid matrix is 0.35-0.45 parts by weight, preferably 0.375-0.40 parts by weight,

the particle size of the active pharmaceutical ingredient satisfies: $1 \ \mu m \leq D_{90} \leq 50 \ \mu m$.

In some embodiments of the first aspect of the present invention, the ophthalmic pharmaceutical composition is an ophthalmic pharmaceutical preparation, preferably an ophthalmic pharmaceutical injection preparation, or a sustained-release insert or implant; the ophthalmic pharmaceutical injection preparation is preferably an intraocular injection preparation, preferably a subconjunctival injection preparation, intravitreal injection preparation, periocular injection preparation, retrobulbar injection preparation, or intracameral injection preparation.

In some embodiments of the first aspect of the present invention, the ophthalmic pharmaceutical composition is in liquid form at room temperature (10-30°C).

In some embodiments of the first aspect of the present invention, the ophthalmic pharmaceutical composition can be filled in a pre-filled syringe. Preferably, the syringe barrel material can be glass (for example, low borosilicate glass, medium borosilicate glass, high borosilicate glass, soda-lime glass, and the like), cyclic olefin copolymer (COC), and/or cyclic olefin polymer (COP). Preferably, the syringe barrel may or may not contain silicone oil.

In some embodiments of the first aspect of the present invention, when filled in a pre-filled syringe, the fill volume of the ophthalmic pharmaceutical composition is 0.01 ml-10 ml. Most preferably, the fill volume range is 0.1 ml-2 ml. For example, the fill volume range can be 0.01, 0.05, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0 ml, or a range defined by any two thereof.

In some embodiments of the first aspect of the present invention, the administration amount of the ophthalmic pharmaceutical composition is $1 \ \mu l$-$100 \ \mu l$. Most preferably, $2$-$20 \ \mu l$. For example, the administration amount can be 1, 2, 3, 4, 5, 6, 7, 8, 9, 10, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 $\mu l$, or a range defined by any two thereof.

In some embodiments of the first aspect of the present invention, the ophthalmic pharmaceutical composition is a sustained-release preparation. Its sustained-release period is 30 days-730 days. Most preferably, the sustained-release period is 60-120 days. For example, the sustained-release period can be 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, 500, 505, 510, 515, 520, 525, 530, 535, 540, 545, 550, 555, 560, 565, 570, 575, 580, 585, 590, 595, 600, 605, 610, 615, 620, 625, 630, 635, 640, 645, 650, 655, 660, 665, 670, 675, 680, 685, 690, 695, 700, 705, 710, 715, 720, 725, 730 days, or a range defined by any two thereof.

In some embodiments of the first aspect of the present invention, the administration frequency of the ophthalmic pharmaceutical composition is once every 30 days-730 days. Most preferably, the administration frequency is once every 60-120 days. For example, the administration frequency can be once every 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, 500, 505, 510, 515, 520, 525, 530, 535, 540, 545, 550, 555, 560, 565, 570, 575, 580, 585, 590, 595, 600, 605, 610, 615, 620, 625, 630, 635, 640, 645, 650, 655, 660, 665, 670, 675, 680, 685, 690, 695, 700, 705, 710, 715, 720, 725, 730 days, or a range defined by any two thereof.

When the ophthalmic pharmaceutical composition of the present invention comprises 0.95-1.25 parts by weight of the liquid matrix and 0.35-0.45 parts by weight of the solid matrix relative to 1 part by weight of the active pharmaceutical ingredient, and simultaneously the active pharmaceutical ingredient satisfies the particle size range: $D_{90} \leq 50 \ \mu m$, the ophthalmic pharmaceutical composition of the present invention meets the required starting force requirement for injection, can be injected into the eye by injection, facilitates injection operation, and can slowly release the active pharmaceutical ingredient in the eye for a long time, and can slowly degrade in the eye. Moreover, the ophthalmic pharmaceutical composition of the present invention has good storage stability.

**Method for Preparing Ophthalmic Pharmaceutical Composition**

The second aspect of the present invention relates to a method for preparing the ophthalmic pharmaceutical composition of the first aspect of the present invention, comprising the steps of:

(1) providing an active pharmaceutical ingredient satisfying the particle size relationship defined in the first aspect of the present invention;
(2) mixing the solid matrix with the liquid matrix, preferably dissolving the solid matrix in the liquid matrix, to obtain a mixed matrix; and
(3) dispersing the active pharmaceutical ingredient provided in step (1) in the mixed matrix obtained in step (2).

In some embodiments of the second aspect of the present invention, the method further comprises a step of pulverizing the active pharmaceutical ingredient before step (1) to provide the active pharmaceutical ingredient having said particle size.

The pulverizing step can be performed by any method known to those skilled in the art that effectively pulverizes the active pharmaceutical ingredient without adversely destroying or denaturing the active pharmaceutical ingredient, for example, the pulverizing can be performed by pulverization methods such as grinding, jet milling, and the like.

In some embodiments of the second aspect of the present invention, the mixing in step (2) is performed under conditions effective to promote dispersion, preferably dissolution, of the solid matrix in the liquid matrix without causing decomposition or denaturation.

For example, in some embodiments of the second aspect of the present invention, the mixing in step (2) can be performed at 20°C-100°C, for example, 20°C, 25°C, 30°C, 40°C, 45°C, 50°C, 55°C, 60°C, 65°C, 70°C, 75°C, 80°C, 85°C, 90°C, 95°C, 100°C.

For example, in some embodiments of the second aspect of the present invention, the mixing in step (2) can optionally be performed in the presence of a solvent to promote dispersion, preferably dissolution, of the solid matrix. The solvent can be appropriately selected according to the polymer used, and can be, for example, ethyl acetate, ethanol, supercritical carbon dioxide, and the like. After the solid matrix is uniformly dispersed, preferably dissolved, the solvent can be removed, for example, by any method known in the art such as heating evaporation, reduced pressure, and the like.

In some embodiments of the second aspect of the present invention, step (3) is performed under conditions effective to disperse the active pharmaceutical ingredient in the mixed matrix without destroying or denaturing the active pharmaceutical ingredient. For example, in accordance with the decomposition or denaturation temperature of the active pharmaceutical ingredient, the dispersion in step (3) can be performed at 30-80°C, for example 40-60°C, for example, 30°C, 40°C, 45°C, 50°C, 55°C, 60°C, 65°C, 70°C, 75°C, 80°C, or a temperature within a range defined by any two thereof.

**Ophthalmic Kit**

The third aspect of the present invention relates to an ophthalmic kit comprising the ophthalmic pharmaceutical composition of the first aspect of the present invention.

In some embodiments of the third aspect of the present invention, the ophthalmic kit comprises one or more of the following i to ii:

> i. the ophthalmic kit further comprises a syringe (for example, a medical syringe);
> ii. the ophthalmic kit is an ophthalmic injection kit.

Preferably, the ophthalmic kit comprises a pre-filled syringe, wherein the ophthalmic pharmaceutical composition is filled in the pre-filled syringe. Preferably, the syringe barrel material can be glass (for example, low borosilicate glass, medium borosilicate glass, high borosilicate glass, soda-lime glass, and the like.), cyclic olefin copolymer (COC), and/or cyclic olefin polymer (COP). Preferably, the syringe barrel may or may not contain silicone oil.

In some embodiments of the third aspect of the present invention, when filled in a pre-filled syringe, the fill volume of the ophthalmic pharmaceutical composition is 0.01 ml-10 ml. Most preferably, the fill volume range is 0.1 ml-2 ml. For example, the fill volume range can be 0.01, 0.05, 0.1, 0.2, 0.3, 0.4, 0.5, 0.6, 0.7, 0.8, 0.9, 1.0, 1.5, 2.0, 2.5, 3.0, 4.0, 5.0, 6.0, 7.0, 8.0, 9.0, 10.0 ml, or a range defined by any two thereof.

In some embodiments of the third aspect of the present invention, when pushing the syringe, with reference to the standard YBB00112004-2015 for pre-filled syringe assemblies, the starting force does not exceed 25 N.

Use

A fourth aspect of the present invention relates to the use of the ophthalmic pharmaceutical composition of the first aspect of the present invention, or the ophthalmic kit of the third aspect of the present invention in the preparation of a medicament for preventing and/or treating an ocular disease.

In some embodiments of the fourth aspect of the present invention, the ocular disease is a posterior segment disease.

In some embodiments of the fourth aspect of the present invention, the ocular disease is selected from one or a mixture of two or more of post-cataract surgery inflammation, glaucoma, uveitis, retinal vein occlusion, retinal artery occlusion, diabetic retinopathy, retinal vasculitis, proliferative vitreoretinopathy, choroidal neovascularization, cystoid macular edema, age-related macular degeneration, vitreomacular adhesion, macular hole, optic neuritis, optic disc edema, optic nerve meningioma, optic nerve glioma, retinoblastoma, and choroidoblastoma.

### EXAMPLES

The embodiments of the present invention will be described in detail below with reference to the examples and the accompanying drawings. However, those skilled in the art will understand that the following examples are only for illustrating the present invention and should not be considered as limiting the scope of the present invention. The materials used in the examples and comparative examples are as follows:

PEG 400: Provided by Nanjing Well Pharmaceutical Co., Ltd., with a number average molecular weight of 380-420. Glycerol: Provided by Taiko Palm-Oleo (Zhangjiagang) Co., Ltd., injection grade. Propylene Glycol: Provided by Hunan Er-Kang Pharmaceutical Co., Ltd.

Glyceryl Triacetate, Diethyl Succinate, Diethyl Tartrate: Provided by Sigma-Aldrich (Shanghai) Trading Co. Ltd.

Polylactic acid-glycolic acid copolymer: Provided by Evonik Specialty Chemicals (Shanghai) Co., Ltd. (Material name: 7525 DLG 2CA, PS5210C), intrinsic viscosity 0.17 dL/g (30°C) and 0.18 dL/g (25°C), weight average molecular weight Mw 16000 when measured in $CHCl_3$, with a number average molecular weight of 9000, the molecular weight distribution (Mw/Mn, also called polydispersity) of 1.9, the weight average molecular weight Mw of 14000 when measured in THF, the number average molecular weight of 8000, the molecular weight distribution of 1.7, wherein the mass ratio of glycolic acid units : lactic acid units is 1:3.

PEG1000: Provided by Sigma-Aldrich (Shanghai) Trading Co. Ltd., number average molecular weight: 950-1050.

PEG20000: Provided by Shanghai Aladdin Biochemical Technology Co., Ltd., with a average number average molecular weight of 20000 (average number average molecular weight range of 18500-22000).

Polyglycolic acid: Provided by Shanghai Aladdin Biochemical Technology Co., Ltd., inherent viscosity 1.4 dl/g (HFIP, 25°C, 0.1g/dl, 1.3-1.6 dl/g).

Polylactic acid: Provided by Shanghai Aladdin Biochemical Technology Co., Ltd., Mw~60000 (molecular weight range of 50000-70000).

Triamcinolone Acetonide, Dexamethasone: Provided by Tianjin Tianyao Pharmaceutical Co., Ltd. Pilocarpine, Brimonidine: Provided by Guangzhou Greensyn Pharmaceutical Co., Ltd. Methotrexate: Provided by Hunan Zhanwang Pharmaceutical Co., Ltd. Cyclosporine: Provided by Zhejiang Ruibang Pharmaceutical Co., Ltd. The respective raw materials (i.e., active pharmaceuticals) were pulverized using a jet mill to prepare raw material particles with corresponding particle sizes $D_{90}$ of 1, 10, 20, 30, 50, and 70 $\mu$m, wherein this particle size was measured using the Laser Scattering with a laser particle size analyzer.

**Examples 1A-5C**

Ophthalmic preparations of Examples 1A-5C were prepared according to the formulations in Table 1.

Table 1

| Ophthalmic Preparation | Pharmaceuti cal (A) | Liquid Matrix (B) | Solid Matrix (C) | A:B:C Mass Ratio | Pharmaceutica 1 Particle Size $D_{90}$ ($\mu$m) |
|---|---|---|---|---|---|
| Example 1A | Triamcinolo ne Acetonide | PEG40 0 | Polylactic acid-glycolic acid copolymer | 1:1.125:0.375 | 1 |
| Example 1B | | | | | 30 |
| Example 1C | | | | | 50 |
| Example 2A | | | | 1:0.95:0.35 | 1 |
| Example 2B | | | | | 30 |
| Example 2C | | | | | 50 |
| Example 3A | | | | 1:0.95:0.45 | 1 |
| Example 3B | | | | | 30 |
| Example 3C | | | | | 50 |
| Example 4A | | | | 1:1.25:0.45 | 1 |
| Example 4B | | | | | 30 |
| Example 4C | | | | | 50 |
| Example 5A | | | | 1:1.25:0.35 | 1 |
| Example 5B | | | | | 30 |
| Example 5C | | | | | 50 |

Each ophthalmic preparation was specifically prepared as follows.

Preparation of Ophthalmic Preparations of Examples 1A, 1B, and 1C

11.25 g of polylactic acid-glycolic acid copolymer was weighed and placed in a beaker. 33.75 g of PEG400 was added thereto, and the mixture was stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparations of Examples 1A, 1B, and 1C.

Preparation of Ophthalmic Preparations of Examples 2A, 2B, and 2C

10.5 g of polylactic acid-glycolic acid copolymer was weighed and placed in a beaker. 28.5 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparations of Examples 2A, 2B, and 2C.

Preparation of Ophthalmic Preparations of Examples 3A, 3B, and 3C

13.5 g of polylactic acid-glycolic acid copolymer was weighed and placed in a beaker. 28.5 g of PEG400 was added thereto.

Stirred at 60°C until the polylactic acid-glycolic acid copolymer was completely dissolved. While stirring, 30 g of triamcinolone acetonide with a pharmaceutical particle size $D_{90}$ of 1 μm, 30 μm, 50 μm, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparations of Examples 3A, 3B, and 3C.

Preparation of Ophthalmic Preparations of Examples 4A, 4B, and 4C

13.5 g of polylactic acid-glycolic acid copolymer was weighed and placed in a beaker. 37.5 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 μm, 30 μm, 50 μm, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparations of Examples 4A, 4B, and 4C.

Preparation of Ophthalmic Preparations of Examples 5A, 5B, and 5C

10.5 g of polylactic acid-glycolic acid copolymer was weighed and placed in a beaker. 37.5 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 μm, 30 μm, 50 μm, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparations of Examples 5A, 5B, and 5C.

**Comparative Examples 1-35**

Ophthalmic preparations of Comparative Examples 1-35 were prepared according to the formulations in Table 2.

Table 2

| Ophthalmic Preparation | Pharmaceutical (A) | Liquid Matrix (B) | Solid Matrix (C) | A:B:C Mass Ratio | Pharmaceutical Particle Size $D_{90}$ (μm) |
|---|---|---|---|---|---|
| Comparative Example 1 | Triamcinolone Acetonide | PEG400 | Polylactic acid-glycolic acid copolymer | 1:1.125:0.3 | 1 |
| Comparative Example 2 | | | | | 30 |
| Comparative Example 3 | | | | | 50 |
| Comparative Example 4 | | | | 1:1.125:0.5 | 1 |
| Comparative Example 5 | | | | | 30 |
| Comparative Example 6 | | | | | 50 |
| Comparative Example 7 | | | | 1:0.9:0.375 | 1 |
| Comparative Example 8 | | | | | 30 |
| Comparative Example 9 | | | | | 50 |
| Comparative Example 10 | | | | 1:1.3:0.375 | 1 |
| Comparative Example 11 | | | | | 30 |
| Comparative Example 12 | | | | | 50 |

(continued)

| Ophthalmic Preparation | Pharmaceutical (A) | Liquid Matrix (B) | Solid Matrix (C) | A:B:C Mass Ratio | Pharmaceutical Particle Size $D_{90}$ ($\mu$m) |
|---|---|---|---|---|---|
| Comparative Example 13 | | | | 1:0.9:0.3 | 1 |
| Comparative Example 14 | | | | | 30 |
| Comparative Example 15 | | | | | 50 |
| Comparative Example 16 | | | | 1:1.3:0.5 | 1 |
| Comparative Example 17 | | | | | 30 |
| Comparative Example 18 | | | | | 50 |
| Comparative Example 19 | | | | 1:0.1:0.1 | 1 |
| Comparative Example 20 | | | | | 30 |
| Comparative Example 21 | | | | | 50 |
| Comparative Example 22 | | | | 1:5:2 | 1 |
| Comparative Example 23 | | | | | 30 |
| Comparative Example 24 | | | | | 50 |
| Comparative Example 25 | | | | 1:3:0.1 | 1 |
| Comparative Example 26 | | | | | 30 |
| Comparative Example 27 | | | | | 50 |
| Comparative Example 28 | | | | 1:0.3:1 | 1 |
| Comparative Example 29 | | | | | 30 |
| Comparative Example 30 | | | | | 50 |
| Comparative Example 31 | | | | 1:0.95:0.35 | 70 |
| Comparative Example 32 | | | | 1:1.125:0.375 | 70 |
| Comparative Example 33 | | | | 1:0.95:0.45 | 70 |
| Comparative Example 34 | | | | 1:1.25:0.45 | 70 |

(continued)

| Ophthalmic Preparation | Pharmaceutical (A) | Liquid Matrix (B) | Solid Matrix (C) | A:B:C Mass Ratio | Pharmaceutical Particle Size $D_{90}$ ($\mu$m) |
|---|---|---|---|---|---|
| Comparative Example 35 | | | | 1:1.25:0.35 | 70 |

Each ophthalmic preparation was specifically prepared as follows.

Preparation of Ophthalmic Preparations of Comparative Examples 1-3
9 g of polylactic acid-glycolic acid copolymer was weighed and placed in a beaker. 33.75 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 1-3.

Preparation of Ophthalmic Preparations of Comparative Examples 4-6
15 g of polylactic acid-glycolic acid copolymer was weighed and placed in a beaker. 33.75 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 4-6.

Preparation of Ophthalmic Preparations of Comparative Examples 7-9
11.25 g of polylactic acid-glycolic acid copolymer was weighed and placed in a beaker. 27 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 7-9.

Preparation of Ophthalmic Preparations of Comparative Examples 10-12
11.25 g of polylactic acid-glycolic acid copolymer was weighed and placed in a beaker. 39 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 10-12.

Preparation of Ophthalmic Preparations of Comparative Examples 13-15
9 g of polylactic acid-glycolic acid copolymer was weighed and placed in a beaker. 27 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 13-15.

Preparation of Ophthalmic Preparations of Comparative Examples 16-18
15 g of polylactic acid-glycolic acid copolymer was weighed and placed in a beaker. 39 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 16-18.

Preparation of Ophthalmic Preparations of Comparative Examples 19-21
3 g of polylactic acid-glycolic acid copolymer was weighed and placed in a beaker. 3 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 19-21.

Preparation of Ophthalmic Preparations of Comparative Examples 22-24
60 g of polylactic acid-glycolic acid copolymer was weighed and placed in a beaker. 150 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 22-24.

Preparation of Ophthalmic Preparations of Comparative Examples 25-27

3 g of polylactic acid-glycolic acid copolymer was weighed and placed in a beaker. 90 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 25-27.

Preparation of Ophthalmic Preparations of Comparative Examples 28-30

30 g of polylactic acid-glycolic acid copolymer was weighed and placed in a beaker. 9 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 1 $\mu$m, 30 $\mu$m, 50 $\mu$m, was added, respectively, and stirring was continued until the mixture was uniform. The mixture was filled into 1 ml pre-filled syringes, with a fill volume of 0.2 mL per syringe, and sterilized by moist heat at 121°C for 15 min to obtain Comparative Examples 28-30.

Preparation of Ophthalmic Preparation of Comparative Example 31

10.5 g of polylactic acid-glycolic acid copolymer was weighed and placed in a beaker. 28.5 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 70 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain Comparative Example 31.

Preparation of Ophthalmic Preparation of Comparative Example 32

11.25 g of polylactic acid-glycolic acid copolymer was weighed and placed in a beaker. 33.75 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 70 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain Comparative Example 32.

Preparation of Ophthalmic Preparation of Comparative Example 33

13.5 g of polylactic acid-glycolic acid copolymer was weighed and placed in a beaker. 28.5 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 70 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain Comparative Example 33.

Preparation of Ophthalmic Preparation of Comparative Example 34

13.5 g of polylactic acid-glycolic acid copolymer was weighed and placed in a beaker. 37.5 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 70 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain Comparative Example 34.

Preparation of Ophthalmic Preparation of Comparative Example 35

10.5 g of polylactic acid-glycolic acid copolymer was weighed and placed in a beaker. 37.5 g of PEG400 was added thereto. Stirred at 60°C until the polylactic acid-glycolic acid copolymer was completely dissolved. While stirring, 30 g of triamcinolone acetonide with a pharmaceutical particle size $D_{90}$ of 70 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain Comparative Example 35.

**Evaluation Example 1: Examination of In Vitro Release Degree and Starting Force of Ophthalmic Preparations**

5 $\mu$L each of the ophthalmic preparations from Examples 1A, 1B, 1C-5A, 5B, 5C and Comparative Examples 1-35 was taken and injected into beakers containing 100 mL of release medium (0.9% sodium chloride solution, pH=7.4, osmotic pressure=290 mOsmol/kg), respectively. The beakers were placed in a water bath shaker, and the temperature was controlled at 37±0.5°C. At different time points, 95 mL of release medium was withdrawn and an equal amount of fresh release medium was replenished. The above release medium was filtered through a 0.22 $\mu$m microporous membrane, and the triamcinolone acetonide content in the release medium was determined by High Performance Liquid Chromatography (HPLC).

Liquid Chromatography testing conditions: Ultimate, XB-C18 column (4.6×250mm, 5$\mu$m); mobile phase: methanol-water (52.5:47.5, v/v); detection wavelength: 240nm; column temperature: 30°C; flow rate: 1.0 mL·min$^{-1}$; injection volume: 20 $\mu$L.

The cumulative pharmaceutical release amount Rn% was calculated according to the following formula:

$$Rn\% = (C_n V_0 + \sum_{n-1}^{n} C_{n-1} V)/M_t \times 100\%$$

Wherein $V_0$ is the volume of the release medium, Cn is the concentration at the nth sampling, V is the sampling volume, Mt is the total pharmaceutical concentration.

One pre-filled finished product each of the ophthalmic preparations from Examples 1A, 1B, 1C-5A, 5B, 5C and Comparative Examples 1-35 was taken and placed in a fixture, the upper and lower limit devices were adjusted, and the test sample was properly clamped. Various test parameters were entered in the test interface for measurement.

Test parameter settings:

Instrument type: Discovery HR-1TFL-IS Electronic Tensile Machine
Control mode: Constant speed
Direction (compression): 2 mm/min
Each sample was tested 5 times, the results were recorded, and the average value was calculated.

The in vitro sustained-release time and starting force of the ophthalmic preparation examples 1A, 1B, 1C-5A, 5B, 5C and comparative examples 1-35 are shown in Table 3.

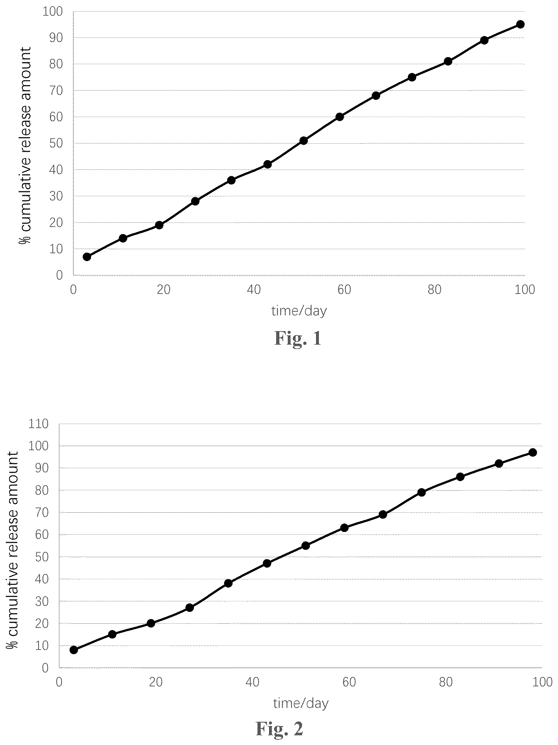

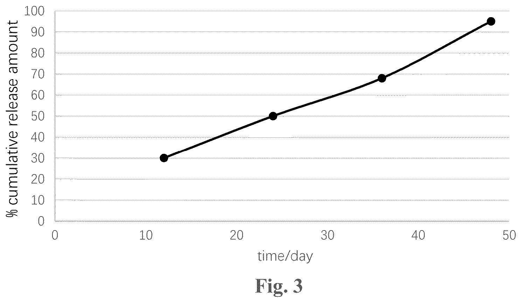

Illustrative graphs of the in vitro release degree of ophthalmic preparation Examples 1B, 2B, and Comparative Example 1 are shown in Figs. 1-3.

Table 3

| Ophthalmic Preparation | Pharmaceutical (A) | Liquid Matrix (B) | Solid Matrix (C) | A:B:C Mass Ratio | Pharmaceutical Particle Size $D_{90}$ ($\mu$m) | Release Time (days) | Starting Force (Fmax, N) |
|---|---|---|---|---|---|---|---|
| Example 1A | Triamcinolone Aceto-nide | PEG4 00 | Polylactic acid-glyco-lic acid co-polymer | 1:1.12 5:0.37 5 | 1 | 93 | 19 |
| Example 1B | | | | | 30 | 99 | 21 |
| Example 1C | | | | | 50 | 105 | 23 |
| Example 2A | | | | 1:0.95: 0.35 | 1 | 91 | 19 |
| Example 2B | | | | | 30 | 98 | 20 |
| Example 2C | | | | | 50 | 107 | 22 |
| Example 3A | | | | 1:0.95: 0.45 | 1 | 98 | 17 |
| Example 3B | | | | | 30 | 105 | 19 |
| Example 3C | | | | | 50 | 112 | 20 |
| Example 4A | | | | 1:1.25: 0.45 | 1 | 95 | 22 |
| Example 4B | | | | | 30 | 103 | 23 |
| Example 4C | | | | | 50 | 109 | 25 |
| Example 5A | | | | 1:1.25: 0.35 | 1 | 80 | 15 |
| Example 5B | | | | | 30 | 90 | 18 |
| Example 5C | | | | | 50 | 100 | 20 |
| Comparative Example 1 | | | | 1:1.12 5:0.3 | 1 | 48 | 21 |
| Comparative Example 2 | | | | | 30 | 56 | 23 |
| Comparative Example 3 | | | | | 50 | 62 | 25 |

(continued)

| Ophthalmic Preparation | Pharmaceutical (A) | Liquid Matrix (B) | Solid Matrix (C) | A:B:C Mass Ratio | Pharmaceutical Particle Size $D_{90}$ ($\mu$m) | Release Time (days) | Starting Force (Fmax, N) |
|---|---|---|---|---|---|---|---|
| Comparative Example 4 | | | | 1:1.12 5:0.5 | 1 | 128 | 40 |
| Comparative Example 5 | | | | | 30 | 133 | 47 |
| Comparative Example 6 | | | | | 50 | 139 | 53 |
| Comparative Example 7 | | | | 1:0.9:0 .375 | 1 | 93 | 45 |
| Comparative Example 8 | | | | | 30 | 105 | 49 |
| Comparative Example 9 | | | | | 50 | 110 | 50 |
| Comparative Example 10 | | | | 1:1.3:0 .375 | 1 | 58 | 20 |
| Comparative Example 11 | | | | | 30 | 62 | 22 |
| Comparative Example 12 | | | | | 50 | 67 | 25 |
| Comparative Example 13 | | | | 1:0.9:0 .3 | 1 | 80 | 32 |
| Comparative Example 14 | | | | | 30 | 90 | 33 |
| Comparative Example 15 | | | | | 50 | 109 | 40 |
| Comparative Example 16 | | | | 1:1.3:0 .5 | 1 | 120 | 40 |
| Comparative Example 17 | | | | | 30 | 126 | 53 |
| Comparative Example 18 | | | | | 50 | 133 | 59 |
| Comparative Example 19 | | | | 1:0.1:0 .1 | 1 | 21 | 28 |
| Comparative Example 20 | | | | | 30 | 28 | 38 |
| Comparative Example 21 | | | | | 50 | 35 | 45 |
| Comparative Example 22 | | | | 1:5:2 | 1 | 134 | 65 |
| Comparative Example 23 | | | | | 30 | 140 | 49 |
| Comparative Example 24 | | | | | 50 | 150 | 67 |

(continued)

| Ophthalmic Preparation | Pharmaceutical (A) | Liquid Matrix (B) | Solid Matrix (C) | A:B:C Mass Ratio | Pharmaceutical Particle Size $D_{90}$ ($\mu$m) | Release Time (days) | Starting Force (Fmax, N) |
|---|---|---|---|---|---|---|---|
| Comparative Example 25 | | | | 1:3:0.1 | 1 | 16 | 12 |
| Comparative Example 26 | | | | | 30 | 21 | 20 |
| Comparative Example 27 | | | | | 50 | 25 | 23 |
| Comparative Example 28 | | | | 1:0.3:1 | 1 | 138 | 38 |
| Comparative Example 29 | | | | | 30 | 140 | 41 |
| Comparative Example 30 | | | | | 50 | 144 | 42 |
| Comparative Example 31 | | | | 1:0.95: 0.35 | 70 | 120 | 45 |
| Comparative Example 32 | | | | 1:1.12 5:0.37 5 | 70 | 118 | 51 |
| Comparative Example 33 | | | | 1:0.95: 0.45 | 70 | 125 | 38 |
| Comparative Example 34 | | | | 1:1.25: 0.45 | 70 | 110 | 40 |
| Comparative Example 35 | | | | 1:1.25: 0.35 | 70 | 115 | 42 |

For the pharmaceutical, other than considering the release time to determine whether the sustained-release effect can be achieved, factors such as whether the starting force meets ergonomic requirements and the syringe's own sealing requirements are also considered. According to the stipulation for the starting force (≤25N) in the "sliding performance" and the stipulation in the "container tightness" that "no leakage shall occur at the contact part between the piston and the syringe barrel when a pressure of 30N is applied" in the quality standard for pre-filled syringe assemblies, the starting force of the preparation was defined as ≤25N. Under this standard, the pharmaceutical is easy to push, and the container sealing is intact. Referring to this principle, all Examples in Table 3 satisfy the starting force ≤25N. Furthermore, the starting forces of Comparative Examples 1-3, 10-12, and 25-27 also do not exceed 25N, but the release times of Comparative Examples 1-3, 10-12, and 25-27 are too fast to achieve a sustained-release effect.

From the results in Table 3, it can be seen that only when the weight ratio of active pharmaceutical : liquid matrix : solid matrix is 1:0.95-1.25:0.35-0.45, and simultaneously the active pharmaceutical particle size range is $D_{90} \leq 50$ $\mu$m, especially 1 $\mu$m $\leq D_{90} \leq 50$ $\mu$m, can the ophthalmic pharmaceutical composition meet the starting force requirement and achieve the sustained-release effect, which can meet the long-term sustained-release effect of the preparation in the eye, simultaneously, is easy to push and facilitates administration operations.

**Evaluation Example 2: Examination of In Vitro Release Degree and Starting Force of Ophthalmic Preparations with Different Formulas**

**Examples 6-10**

Referring to the preparation method of the ophthalmic preparations of Examples 1A-5C, the ophthalmic preparations of Examples 6-10 were prepared according to the formulations in Table 4.

Table 4

| Ophthalmic Preparation | Pharmaceutical (A) | Liquid Matrix (B) | Solid Matrix (C) | A:B:C Mass Ratio | Pharmaceutical Particle Size $D_{90}$ ($\mu$m) |
|---|---|---|---|---|---|
| Example 6 | Dexamethasone | Glyceryl Triacetate | PEG20000 | 1:1:0.4 | 30 |
| Example 7 | Pilocarpine | Propylene Glycol | Polyglycolic Acid | 1:1.25:0.35 | 20 |
| Example 8 | Methotrexate | Glycerol | Polylactic Acid | 1:1.1:0.4 | 1 |
| Example 9 | Cyclosporine | Diethyl Succinate | PEG1000 | 1:0.95:0.45 | 10 |
| Example 10 | Brimonidine | Diethyl Tartrate | Polylactic acid-glycolic acid copolymer | 1:0.95:0.35 | 50 |

Each ophthalmic preparation was specifically prepared as follows.

Preparation of Ophthalmic Preparation of Example 6

12 g of PEG20000 was weighed and placed in a beaker. 30 g of glyceryl triacetate was added thereto. Stirred at 60°C until the PEG20000 was completely dissolved. While stirring, 30 g of dexamethasone with a pharmaceutical particle size $D_{90}$ of 30 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparation of Example 6.

Preparation of Ophthalmic Preparation of Example 7

10.5 g of polyglycolic acid was weighed and placed in a beaker. 37.5 g of propylene glycol was added thereto. Stirred at 60°C until the polyglycolic acid was completely dissolved. While stirring, 30 g of pilocarpine with a pharmaceutical particle size $D_{90}$ of 20 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparation of Example 7.

Preparation of Ophthalmic Preparation of Example 8

12 g of polylactic acid was weighed and placed in a beaker. 33 g of glycerol was added thereto. Stirred at 60°C until the polylactic acid was completely dissolved. While stirring, 30 g of methotrexate with a pharmaceutical particle size $D_{90}$ of 1 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparation of Example 8.

Preparation of Ophthalmic Preparation of Example 9

13.5 g of PEG1000 was weighed and placed in a beaker. 28.5 g of diethyl succinate was added thereto. Stirred at 60°C until the PEG1000 was completely dissolved. While stirring, 30 g of cyclosporine with a pharmaceutical particle size $D_{90}$ of 10 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparation of Example 9.

Preparation of Ophthalmic Preparation of Example 10

10.5 g of polylactic acid-glycolic acid copolymer was weighed and placed in a beaker. 28.5 g of diethyl tartrate was added thereto. Stirred at 60°C until the polylactic acid-glycolic acid copolymer was completely dissolved. While stirring, 30 g of brimonidine with a pharmaceutical particle size $D_{90}$ of 50 $\mu$m was added, and stirring was continued until the mixture was uniform. The mixture was filled into 1 ml pre-filled syringes, with a fill volume of 0.2 mL per syringe, and sterilized by moist heat at 121°C for 15 min to obtain the ophthalmic preparation of Example 10.

The in vitro release degree and starting force of the ophthalmic preparations were examined according to the method in Evaluation Example 1, and the results are shown in Table 5.

Table 5

| Ophthalmic Preparation | Release Time (days) | Starting Force (Fmax, N) |
|---|---|---|
| Example 6 | 95 | 22 |
| Example 7 | 85 | 17 |
| Example 8 | 88 | 20 |

(continued)

| Ophthalmic Preparation | Release Time (days) | Starting Force (Fmax, N) |
|---|---|---|
| Example 9 | 100 | 18 |
| Example 10 | 104 | 23 |

As can be seen from the above table, the sustained-release times of Examples 6-10 are in the range of 85-104 days and the starting forces are in the range of 17-23 N, which are comparable to the effects of Examples 1A-5C. This indicates that under the condition of adopting different active ingredients, liquid matrices, or solid matrices, when the weight ratio of the three satisfies the active pharmaceutical ingredient being 1 part by weight, the liquid matrix being 0.95-1.25 parts by weight, and the solid matrix being 0.35-0.45 parts by weight, and the particle size of the active ingredient satisfies $D_{90} \leq 50\ \mu m$, especially $1\ \mu m \leq D_{90} \leq 50\ \mu m$, the optimal effects of slow release and convenient injection can be achieved.

**Evaluation Example 3: Examination of Dispersion Uniformity of Ophthalmic Preparations**

The ophthalmic preparations from Examples 1A, 1B, 1C-5A, 5B, 5C and Comparative Examples 1-35 were taken, and the dispersion uniformity of each preparation was examined after stability testing.

Dispersion uniformity test: The samples were placed at 60°C and under illumination of (4500±500) Lx for 10 days, and sampling was taken on the 5th and 10th days, respectively. Low-temperature cycle test: Each cycle involved firstly placing at 2~8°C for 2 days, then placing at 40°C for 2 days, sampling and testing. This was recorded as one cycle, and three consecutive cycles were performed. The evaluation index for the preparation: The appearance was examined to see if the state of the pharmaceutical liquid changed before and after the dispersion uniformity test, and whether stratification, aggregation, or caking occurred.

The dispersion uniformity results of the ophthalmic preparation examples 1A, 1B, 1C-5A, 5B, 5C and comparative examples 1-35 are shown in Table 6.

Table 6

| Ophthalmic Preparation | Pharmaceutical (A) | Liquid Matrix (B) | Solid Matrix (C) | A:B:C Mass Ratio | Pharmaceutical Particle Size $D_{90}$ (μm) | Appearance |
|---|---|---|---|---|---|---|
| Example 1A | Triamcinolone Acetonide | PEG40 0 | Polylactic acid-glycolic acid copolymer | 1:1.12 5:0.37 5 | 1 | The pharmaceutical liquid is uniformly dispersed |
| Example 1B | | | | | 30 | The pharmaceutical liquid is uniformly dispersed |
| Example 1C | | | | | 50 | The pharmaceutical liquid is uniformly dispersed |
| Example 2A | | | | 1:0.95: 0.35 | 1 | The pharmaceutical liquid is uniformly dispersed |
| Example 2B | | | | | 30 | The pharmaceutical liquid is uniformly dispersed |
| Example 2C | | | | | 50 | The pharmaceutical liquid is uniformly dispersed |

(continued)

| Ophthalmic Preparation | Pharmaceutical (A) | Liquid Matrix (B) | Solid Matrix (C) | A:B:C Mass Ratio | Pharmaceutical Particle Size $D_{90}$ (μm) | Appearance |
|---|---|---|---|---|---|---|
| Example 3A | | | | 1:0.95: 0.45 | 1 | The pharmaceutical liquid is uniformly dispersed |
| Example 3B | | | | | 30 | The pharmaceutical liquid is uniformly dispersed |
| Example 3C | | | | | 50 | The pharmaceutical liquid is uniformly dispersed |
| Example 4A | | | | 1:1.25: 0.45 | 1 | The pharmaceutical liquid is uniformly dispersed |
| Example 4B | | | | | 30 | The pharmaceutical liquid is uniformly dispersed |
| Example 4C | | | | | 50 | The pharmaceutical liquid is uniformly dispersed |
| Example 5A | | | | 1:1.25: 0.35 | 1 | The pharmaceutical liquid is uniformly dispersed |
| Example 5B | | | | | 30 | The pharmaceutical liquid is uniformly dispersed |
| Example 5C | | | | | 50 | The pharmaceutical liquid is uniformly dispersed |
| Comparative Example 1 | | | | 1:1.12 5:0.3 | 1 | The pharmaceutical liquid is uniformly dispersed |
| Comparative Example 2 | | | | | 30 | The pharmaceutical liquid is uniformly dispersed |
| Comparative Example 3 | | | | | 50 | The pharmaceutical liquid is uniformly dispersed |
| Comparative Example 4 | | | | 1:1.12 5:0.5 | 1 | The pharmaceutical liquid is uniformly dispersed |
| Comparative Example 5 | | | | | 30 | The pharmaceutical liquid is uniformly dispersed |
| Comparative Example 6 | | | | | 50 | The pharmaceutical liquid is uniformly dispersed |

(continued)

| Ophthalmic Preparation | Pharmaceutical (A) | Liquid Matrix (B) | Solid Matrix (C) | A:B:C Mass Ratio | Pharmaceutical Particle Size $D_{90}$ ($\mu$m) | Appearance |
|---|---|---|---|---|---|---|
| Comparative Example 7 | | | | 1:0.9:0 .375 | 1 | The pharmaceutical liquid is uniformly dispersed |
| Comparative Example 8 | | | | | 30 | The pharmaceutical liquid is uniformly dispersed |
| Comparative Example 9 | | | | | 50 | The pharmaceutical liquid is uniformly dispersed |
| Comparative Example 10 | | | | 1:1.3:0 .375 | 1 | The pharmaceutical liquid is uniformly dispersed |
| Comparative Example 11 | | | | | 30 | The pharmaceutical liquid is uniformly dispersed |
| Comparative Example 12 | | | | | 50 | The pharmaceutical liquid is uniformly dispersed |
| Comparative Example 13 | | | | 1:0.9:0 .3 | 1 | The pharmaceutical liquid is uniformly dispersed |
| Comparative Example 14 | | | | | 30 | The pharmaceutical liquid is uniformly dispersed |
| Comparative Example 15 | | | | | 50 | The pharmaceutical liquid is uniformly dispersed |
| Comparative Example 16 | | | | 1:1.3:0 .5 | 1 | The pharmaceutical liquid is uniformly dispersed |
| Comparative Example 17 | | | | | 30 | The pharmaceutical liquid is uniformly dispersed |
| Comparative Example 18 | | | | | 50 | The pharmaceutical liquid is uniformly dispersed |
| Comparative Example 19 | | | | 1:0.1:0 .1 | 1 | The pharmaceutical liquid has caking |
| Comparative Example 20 | | | | | 30 | The pharmaceutical liquid has caking |
| Comparative Example 21 | | | | | 50 | The pharmaceutical liquid has caking |

(continued)

| Ophthalmic Preparation | Pharmaceutical (A) | Liquid Matrix (B) | Solid Matrix (C) | A:B:C Mass Ratio | Pharmaceutical Particle Size $D_{90}$ (μm) | Appearance |
|---|---|---|---|---|---|---|
| Comparative Example 22 | | | | 1:5:2 | 1 | The pharmaceutical liquid is uniformly dispersed |
| Comparative Example 23 | | | | | 30 | The pharmaceutical liquid is uniformly dispersed |
| Comparative Example 24 | | | | | 50 | The pharmaceutical liquid is uniformly dispersed |
| Comparative Example 25 | | | | 1:3:0.1 | 1 | The pharmaceutical liquid has stratification |
| Comparative Example 26 | | | | | 30 | The pharmaceutical liquid has stratification |
| Comparative Example 27 | | | | | 50 | The pharmaceutical liquid has stratification |
| Comparative Example 28 | | | | 1:0.3:1 | 1 | The pharmaceutical liquid is uniformly dispersed |
| Comparative Example 29 | | | | | 30 | The pharmaceutical liquid is uniformly dispersed |
| Comparative Example 30 | | | | | 50 | The pharmaceutical liquid is uniformly dispersed |
| Comparative Example 31 | | | | 1:0.95: 0.35 | 70 | The pharmaceutical liquid has stratification |
| Comparative Example 32 | | | | 1:1.12 5:0.37 5 | 70 | The pharmaceutical liquid has stratification |
| Comparative Example 33 | | | | 1:0.95: 0.45 | 70 | The pharmaceutical liquid has stratification |
| Comparative Example 34 | | | | 1:1.25: 0.45 | 70 | The pharmaceutical liquid has stratification |
| Comparative Example 35 | | | | 1:1.25: 0.35 | 70 | The pharmaceutical liquid has stratification |

Note: "The pharmaceutical liquid is uniformly dispersed" means the active pharmaceutical ingredient is uniformly dispersed in the matrix without phenomena such as stratification, precipitation, caking or the like. "The pharmaceutical liquid has stratification" means the pharmaceutical is not uniformly dispersed, and a clear liquid appears in the upper layer of the pharmaceutical liquid. "The pharmaceutical liquid has caking" means the pharmaceutical is not uniformly dispersed, and phenomena of aggregation or caking of the pharmaceutical occurs.

# EP 4 710 918 A1

From the results in Table 6, it can be seen that when the weight ratio of the active ingredient, liquid matrix, and solid matrix satisfies the active pharmaceutical ingredient being 1 part by weight, the liquid matrix being 0.95-1.25 parts by weight, and the solid matrix being 0.35-0.45 parts by weight, and simultaneously the active pharmaceutical ingredient particle size range is $D_{90} \leq 50 \ \mu m$, the pharmaceutical liquid has good dispersion uniformity, without phenomena of stratification, precipitation, or caking; it can meet the long-term sustained-release effect of the preparation in the eye, simultaneously, is easy to push, facilitates administration operations, and has good dispersion uniformity. When the particle size $D_{90}$ of the active pharmaceutical ingredient is 70 $\mu m$ (for example, Comparative Examples 31-35), the prepared pharmaceutical liquid showed stratification phenomenon; when the particle size $D_{90}$ of the active pharmaceutical ingredient is $\leq 50 \ \mu m$, if the amounts of both the liquid matrix and the solid matrix are too low (for example, Comparative Examples 19-21), the prepared pharmaceutical liquid showed caking phenomenon; if the liquid matrix is too much and the solid matrix is too little (for example, Comparative Examples 25-27), the prepared pharmaceutical liquid showed stratification phenomenon.

The above examples are only used to illustrate the technical solutions of the present invention and not to limit them; although the present invention has been described in detail with reference to the preferred embodiments, those of ordinary skill in the art should understand that the specific embodiments of the present invention can still be modified or some technical features can be equivalently replaced; without departing from the spirit of the technical solutions of the present invention, all should be covered within the scope of the technical solutions claimed in the present invention.

## Claims

1. An ophthalmic pharmaceutical composition comprising, or consisting of the following components:

   | | |
   |---|---|
   | active pharmaceutical ingredient | 1 part by weight, |
   | liquid matrix | 0.95-1.25 parts by weight, |
   | solid matrix | 0.35-0.45 parts by weight, |

   wherein the particle size of the active pharmaceutical ingredient satisfies: $D_{90} \leq 50 \ \mu m$.

2. The ophthalmic pharmaceutical composition according to claim 1, wherein:
   the particle size of the active pharmaceutical ingredient satisfies: $1 \ \mu m \leq D_{90} \leq 50 \ \mu m$.

3. The ophthalmic pharmaceutical composition according to any one of claims 1-2, wherein:

   the active pharmaceutical ingredient is selected from one or a mixture of two or more of antitumor drugs, anti-inflammatory drugs, immunosuppressants, neovascularization inhibitors, and glaucoma therapeutic drugs;
   preferably, the active pharmaceutical ingredient is selected from one or a mixture of two or more of chlorambucil, melphalan, cyclophosphamide, ifosfamide, carmustine, lomustine, semustine, chlorozotocin, cisplatin, carboplatin, oxaliplatin, fluorouracil, mitoxantrone, irinotecan, topotecan, vinblastine, vincristine, vindesine, vinorelbine, etoposide, teniposide, paclitaxel, docetaxel, bleomycin, methotrexate, gemcitabine, capecitabine, hydroxyurea, mitomycin, gefitinib, sunitinib, dexamethasone, dexamethasone acetate, prednisone, prednisone acetate, fluocinolone, fluocinolone acetonide, triamcinolone acetonide, methylprednisolone, methylprednisolone aceponate, halobetasol propionate, cortisone, hydrocortisone, cyclosporine, rapamycin, tacrolimus, mycophenolate mofetil, fujimycin, mizoribine, sulfasalazine, azathioprine, methotrexate, bevacizumab, ranibizumab, pegaptanib sodium, aflibercept, latanoprost, travoprost, bimatoprost, bimatoprost, tafluprost, pilocarpine, atropine, hyoscyamine, betaxolol, metoprolol, bupranolol, metipranolol, propranolol, timolol, befunolol, acetazolamide, dorzolamide, brinzolamide, apraclonidine, brimonidine, dipivefrin, guanethidine, dapiprazole, dasatinib, and pharmaceutically acceptable salts thereof;
   preferably, the active pharmaceutical ingredient is selected from one or a mixture of two or more of dexamethasone, latanoprost, dasatinib, triamcinolone acetonide, pilocarpine, methotrexate, cyclosporine, brimonidine, and pharmaceutically acceptable salts thereof.

4. The ophthalmic pharmaceutical composition according to any one of claims 1-3, wherein:

   the liquid matrix is a biodegradable liquid matrix, preferably the liquid matrix is one or a mixture of two or more of diethyl succinate, diethyl tartrate, dimethyl glutarate, glyceryl triacetate, polyethylene glycol, glycerol, propylene glycol, and mixtures thereof, preferably wherein the polyethylene glycol of the liquid matrix is one or more of polyethylene glycols having a number average molecular weight of 70-750, for example, one or a mixture of two or more of PEG-200, PEG-300, PEG-400, and PEG-600;
preferably, the liquid matrix is polyethylene glycol, preferably polyethylene glycol PEG-400.

5. The ophthalmic pharmaceutical composition according to any one of claims 1-4, wherein:

the solid matrix is a biodegradable solid matrix, preferably the solid matrix is one or a mixture of two or more of polyethylene glycol, hydroxycarboxylate polymers, preferably the solid matrix is one or a mixture of two or more of polyethylene glycol, polylactic acid, polyglycolic acid, polylactic acid-glycolic acid copolymer, preferably wherein the polyethylene glycol of the solid matrix is one or a mixture of two or more of polyethylene glycols having a number average molecular weight of 800-20,000, for example, one or a mixture of two or more of PEG-800, PEG-1000, PEG-1500, PEG-2000, PEG-4000, PEG-6000, PEG-8000, PEG-10000, and PEG-20000;
preferably, the solid matrix is polylactic acid-glycolic acid copolymer;
preferably, in the polylactic acid-glycolic acid copolymer, the mass ratio of units derived from glycolic acid to units derived from lactic acid is 1:0.01-100, preferably 1:0.02-50, preferably 1:0.05-20, preferably 1:0.1-10, preferably 1:0.2-5, preferably 1:0.5-4, preferably 1:1-3;
preferably, the hydroxycarboxylate polymer of the solid matrix has a weight average molecular weight of 1,000-600,000, preferably 5,000-200,000, for example 10,000-20,000; preferably, the molecular weight distribution Mw/Mn of the solid matrix is 1.0-10.0, for example 1.5-5.0.

6. The ophthalmic pharmaceutical composition according to any one of claims 1-5, wherein:

the active pharmaceutical ingredient is triamcinolone acetonide,
the liquid matrix is polyethylene glycol PEG-400;
the solid matrix is polylactic acid-glycolic acid copolymer.

7. The ophthalmic pharmaceutical composition according to any one of claims 1-6, wherein:

the ophthalmic pharmaceutical composition is an ophthalmic pharmaceutical preparation, preferably an ophthalmic pharmaceutical injection preparation, or a sustained-release insert or implant, the ophthalmic pharmaceutical injection preparation is preferably an intraocular injection preparation, preferably a subconjunctival injection preparation, intravitreal injection preparation, periocular injection preparation, retrobulbar injection preparation, or intracameral injection preparation;
preferably, the ophthalmic pharmaceutical preparation is a sustained-release preparation;
preferably, the ophthalmic pharmaceutical composition is filled in a pre-filled syringe, preferably the fill volume of the ophthalmic pharmaceutical composition is 0.01 ml-10 ml, preferably 0.1 ml-2 ml;
preferably, the administration amount of the ophthalmic pharmaceutical composition is 1 $\mu$l-100 $\mu$l, preferably 2-20 $\mu$l;
preferably, the sustained-release period of the ophthalmic pharmaceutical composition is 30 days-730 days, preferably 60-120 days;
preferably, the administration frequency of the ophthalmic pharmaceutical composition is once every 30 days-730 days, preferably once every 60-120 days;
preferably, the ophthalmic pharmaceutical composition is in liquid form at room temperature.

8. A method for preparing the ophthalmic pharmaceutical composition according to any one of claims 1-7, comprising:

(1) providing an active pharmaceutical ingredient satisfying the particle size relationship defined in any one of claims 1-2;
(2) mixing the solid matrix with the liquid matrix, preferably dissolving the solid matrix in the liquid matrix, to obtain a mixed matrix; and
(3) dispersing the active pharmaceutical ingredient provided in step (1) in the mixed matrix obtained in step (2);

optionally, the method further comprises a step of pulverizing the active pharmaceutical ingredient before step (1).

9. An ophthalmic kit comprising the ophthalmic pharmaceutical composition according to any one of claims 1-7, preferably, the ophthalmic kit comprises a pre-filled syringe, wherein the ophthalmic pharmaceutical composition is filled in the pre-filled syringe, preferably the fill volume of the ophthalmic pharmaceutical composition is 0.01 ml-10 ml, preferably 0.1 ml-2 ml.

10. The use of the ophthalmic pharmaceutical composition according to any one of claims 1-7 or the ophthalmic kit according to claim 9 in the preparation of a medicament for preventing and/or treating an ocular disease.

Fig. 1

Fig. 2

Fig. 3

## INTERNATIONAL SEARCH REPORT

| International application No. |
|---|
| **PCT/CN2024/103354** |

**A. CLASSIFICATION OF SUBJECT MATTER**

A61K 9/00(2006.01)i; A61K47/34(2017.01)i; A61K 47/10(2017.01)i; A61K 45/00(2006.01)i; A61P 27/02(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNKI, CNABS, CNTXT, DWPI, SIPOABS, ENTXTC, STN, 百度, BAIDU: 沈阳兴齐, 聚乳酸-羟基乙酸共聚物, 聚(丙交酯-乙交酯)共聚物, 聚乳酸-乙醇酸共聚物, 聚乳酸, 聚羟基乙酸, 聚乙二醇, 甘油三醋酸酯, 丙二醇, 甘油, 丁二酸二乙酯, 酒石酸二乙酯, 注射, 缓释, 眼, 曲安奈德, PLGA, PLA, PEG20000, PEG1000, PEG400, poly(lactic-co-glycolic acid), polylactide, polyethylene glycol, triamcinolone, sustained-release, inject, ophthalmic, ocular

**C. DOCUMENTS CONSIDERED TO BE RELEVANT**

| Category* | Citation of document, with indication, where appropriate, of the relevant passages | Relevant to claim No. |
|---|---|---|
| Y | CN 107595765 A (SHENYANG SINQI PHARMACEUTICAL CO., LTD.) 19 January 2018 (2018-01-19) <br> claims 1 and 10, and description, paragraphs [0029]-[0034] and [0065]-[0066] | 1-10 |
| Y | CN 110475545 A (W.L. GORE & ASSOCIATES, INC.) 19 November 2019 (2019-11-19) <br> description, paragraphs [0004], [0028], and [0076]-[0085] | 1-10 |
| Y | CN 109549918 A (SHENYANG SINQI PHARMACEUTICAL CO., LTD.) 02 April 2019 (2019-04-02) <br> claims 1, 3-7, and 10, and description, paragraphs [0142]-[0189] | 1-10 |
| A | CN 101862455 A (PSIVIDA US INC.) 20 October 2010 (2010-10-20) <br> claims 1-7 and 10, and description, paragraphs [0028], [0039], [0045], and [0049]-[0070] | 1-10 |
| A | CN 101827523 A (MACUSIGHT INC.) 08 September 2010 (2010-09-08) <br> claim 96, and description, paragraph [615] | 1-10 |
| A | CN 101437478 A (QLT USA INC.) 20 May 2009 (2009-05-20) <br> claims 1, 9, 29, 37-38, 46, 49, and 93-94, and description, embodiments 1-2 | 1-10 |

☐ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **28 September 2024** | **09 October 2024** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| International application No. |
| --- |
| **PCT/CN2024/103354** |

| Patent document cited in search report | | | Publication date (day/month/year) | Patent family member(s) | | | Publication date (day/month/year) |
| --- | --- | --- | --- | --- | --- | --- | --- |
| CN | 107595765 | A | 19 January 2018 | ES | 2945159 | T3 | 28 June 2023 |
| | | | | JP | 2020535220 | A | 03 December 2020 |
| | | | | JP | 7173666 | B2 | 16 November 2022 |
| | | | | EP | 3685828 | A1 | 29 July 2020 |
| | | | | EP | 3685828 | A4 | 11 August 2021 |
| | | | | EP | 3685828 | B1 | 19 April 2023 |
| | | | | US | 2020215079 | A1 | 09 July 2020 |
| | | | | WO | 2019057110 | A1 | 28 March 2019 |
| | | | | WO | 2019056751 | A1 | 28 March 2019 |
| | | | | JP | 2022180505 | A | 06 December 2022 |
| | | | | JP | 7519413 | B2 | 19 July 2024 |
| | | | | EP | 4074306 | A1 | 19 October 2022 |
| CN | 110475545 | A | 19 November 2019 | AU | 2020203725 | A1 | 25 June 2020 |
| | | | | WO | 2018183224 | A2 | 04 October 2018 |
| | | | | WO | 2018183224 | A3 | 08 November 2018 |
| | | | | KR | 20190126175 | A | 08 November 2019 |
| | | | | KR | 102345038 | B1 | 29 December 2021 |
| | | | | JP | 2021193153 | A | 23 December 2021 |
| | | | | EP | 3838262 | A1 | 23 June 2021 |
| | | | | AU | 2021277631 | A1 | 23 December 2021 |
| | | | | AU | 2021277631 | B2 | 30 March 2023 |
| | | | | AU | 2018243796 | A1 | 12 September 2019 |
| | | | | AU | 2018243796 | B2 | 05 March 2020 |
| | | | | AU | 2018243796 | B8 | 09 July 2020 |
| | | | | CA | 3055429 | A1 | 04 October 2018 |
| | | | | CA | 3055429 | C | 07 December 2021 |
| | | | | EP | 3600248 | A2 | 05 February 2020 |
| | | | | EP | 3600248 | B1 | 16 June 2021 |
| | | | | CA | 3135191 | A1 | 04 October 2018 |
| | | | | CA | 3135191 | C | 16 January 2024 |
| | | | | US | 2018271779 | A1 | 27 September 2018 |
| | | | | US | 11752099 | B2 | 12 September 2023 |
| | | | | KR | 20220020281 | A | 18 February 2022 |
| | | | | KR | 102548649 | B1 | 27 June 2023 |
| | | | | JP | 2020512339 | A | 23 April 2020 |
| | | | | JP | 6955577 | B2 | 27 October 2021 |
| CN | 109549918 | A | 02 April 2019 | None | | | |
| CN | 101862455 | A | 20 October 2010 | CY | 1108685 | T1 | 09 April 2014 |
| | | | | TW | 200514581 | A | 01 May 2005 |
| | | | | TWI | 377958 | B | 01 December 2012 |
| | | | | US | 2013101656 | A1 | 25 April 2013 |
| | | | | SI | 1635875 | T1 | 30 April 2009 |
| | | | | EP | 2044959 | A1 | 08 April 2009 |
| | | | | CA | 2530136 | A1 | 13 January 2005 |
| | | | | CA | 2530136 | C | 16 October 2012 |
| | | | | DE | 602004016995 | D1 | 20 November 2008 |
| | | | | WO | 2005002625 | A2 | 13 January 2005 |
| | | | | WO | 2005002625 | A3 | 21 April 2005 |
| | | | | ES | 2315680 | T3 | 01 April 2009 |
| | | | | ATE | 410186 | T1 | 15 October 2008 |

Form PCT/ISA/210 (patent family annex) (July 2022)

**INTERNATIONAL SEARCH REPORT**
Information on patent family members

International application No.

**PCT/CN2024/103354**

| Patent document cited in search report | | | Publication date (day/month/year) | Patent family member(s) | | | Publication date (day/month/year) |
|---|---|---|---|---|---|---|---|
| | | | | JP | 2007524628 | A | 30 August 2007 |
| | | | | JP | 5229768 | B2 | 03 July 2013 |
| | | | | EP | 1635875 | A2 | 22 March 2006 |
| | | | | EP | 1635875 | B1 | 08 October 2008 |
| | | | | EP | 1635875 | B8 | 24 December 2008 |
| | | | | PL | 1635875 | T3 | 31 March 2009 |
| | | | | AR | 044926 | A1 | 12 October 2005 |
| | | | | DK | 1635875 | T3 | 12 January 2009 |
| | | | | US | 2005048123 | A1 | 03 March 2005 |
| | | | | US | 2014336278 | A1 | 13 November 2014 |
| | | | | US | 9566336 | B2 | 14 February 2017 |
| | | | | PT | 1635875 | E | 09 December 2008 |
| | | | | US | 2011165242 | A1 | 07 July 2011 |
| CN | 101827523 | A | 08 September 2010 | JP | 2010536797 | A | 02 December 2010 |
| | | | | WO | 2009023877 | A2 | 19 February 2009 |
| | | | | WO | 2009023877 | A3 | 09 April 2009 |
| | | | | ES | 2507078 | T3 | 14 October 2014 |
| | | | | EP | 2187743 | A2 | 26 May 2010 |
| | | | | EP | 2187743 | A4 | 27 October 2010 |
| | | | | EP | 2187743 | B1 | 30 July 2014 |
| | | | | KR | 20100055482 | A | 26 May 2010 |
| CN | 101437478 | A | 20 May 2009 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310821685